(19) Europäisches Patentamt / European Patent Office / Office européen des brevets

(11) **EP 4 342 362 B1**

(12) **EUROPEAN PATENT SPECIFICATION**

(45) Date of publication and mention
of the grant of the patent:
**25.03.2026 Bulletin 2026/13**

(21) Application number: **21940740.0**

(22) Date of filing: **18.05.2021**

(51) International Patent Classification (IPC):
***A61B 3/16*** (2006.01)

(52) Cooperative Patent Classification (CPC):
**A61B 3/16; A61B 3/10; A61B 3/1005; A61B 3/165**

(86) International application number:
**PCT/JP2021/018883**

(87) International publication number:
**WO 2022/244123 (24.11.2022 Gazette 2022/47)**

(54) **NON-CONTACT TYPE EYEBALL PHYSICAL PROPERTY MEASUREMENT DEVICE**

VORRICHTUNG ZUR KONTAKTLOSEN MESSUNG PHYSIKALISCHER EIGENSCHAFTEN DES AUGAPFELS

DISPOSITIF DE MESURE DE PROPRIÉTÉ PHYSIQUE DE GLOBE OCULAIRE DE TYPE SANS CONTACT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.03.2024 Bulletin 2024/13**

(73) Proprietor: **Quovisu LLC**
**Nagoya-shi Aichi 462-0023 (JP)**

(72) Inventor: **KATO Chihiro**
**Nagoya-shi Aichi 462-0023 (JP)**

(74) Representative: **Cabinet Chaillot**
**16/20, avenue de l'Agent Sarre**
**B.P. 74**
**92703 Colombes Cedex (FR)**

(56) References cited:
JP-A- 2015 092 980    JP-A- 2016 518 909
JP-A- 2018 529 419    US-A- 5 148 807
US-A- 5 828 454    US-A1- 2003 078 486

US-A1- 2004 260 168    US-A1- 2017 280 998
US-A1- 2018 116 512    US-A1- 2018 193 194

• MONROY FRANCISCO ET AL: "Direct Experimental Observation of the Crossover from Capillary to Elastic Surface Waves on Soft Gels", vol. 81, no. 15, 1 October 1998 (1998-10-01), US, pages 3167 - 3170, XP093173230, ISSN: 0031-9007, Retrieved from the Internet <URL:https://journals.aps.org/prl/pdf/10.1103/PhysRevLett.81.3167> DOI: 10.1103/PhysRevLett.81.3167
• HAN ZHAOLONG ET AL: "Optical coherence elastography assessment of corneal viscoelasticity with a modified Rayleigh-Lamb wave model", JOURNAL OF THE MECHANICAL BEHAVIOR OF BIOMEDICAL MATERIALS, ELSEVIER, AMSTERDAM, NL, vol. 66, 5 November 2016 (2016-11-05), pages 87 - 94, XP029855074, ISSN: 1751-6161, DOI: 10.1016/J.JMBBM.2016.11.004

## Description

### Field of the Invention

This invention relates to an ocular physical property measuring device that measures physical properties of an eyeball in a non-contact manner, and particularly relates to an ocular physical property measuring device that can measure physical properties such as intraocular pressure of an eyeball, material mechanical properties of the surface tissue of an eyeball and so on.

### Background Art

Conventionally, as a non-contact type measuring device for intraocular pressure, an air flow is blown onto the eyeball to deform the cornea with non-contact, and the air jet pressure of the air flow (Air Puff) is plotted to measure the air jet pressure when the cornea becomes flat. Then, the correlation between this air jet pressure and a comparative measurement value measured using a contact applanation tonometer used as a standard intraocular pressure measurement, is calculated. As a result, it is possible to obtain intraocular pressure value.

For example, in order to measure the mechanical characteristics of the cornea of the eye to be examined and to measure the intraocular pressure, the cornea is pressed inward with an air puff to deform it, thereby forming a temporary flattened shape of the cornea. This flattened point is called the detected first applanation point. After the cornea reaches its maximum deformation due to further depression, it passes through the flat plane again in the process of returning to its original shape, and this is called the second applanation point.

In Patent Reference 1, during this corneal shape deforming process, the air jet pressure is plotted over time, the air jet pressure at the applanation point is measured, and the intraocular pressure is determined from the air jet pressure at the first and second applanation points. In this way, intraocular pressure measurement has been proposed in which the influence of measured values by corneal stiffness is reduced.

Furthermore, in Patent Reference 2, the corneal flattening process is captured by a corneal tomogram using Scheimpflug array illumination and an imaging camera, and the corneal applanation radius and free vibration are measured. In this way, intraocular pressure measurement, which reduces the influence of measured values by corneal stiffness, and a system, which analyzes the mechanical material properties of the cornea, have been proposed.

In addition, as a non-contact tonometer method, several techniques as an ultrasonic (acoustic radiation pressure) non-contact tonometer that measures intraocular pressure by irradiating the cornea with ultrasonic waves and deforming or vibrating the cornea due to the sound pressure, have been proposed. For example, Patent Reference 3 proposes a system that deforms the cornea using acoustic radiation pressure generated by ultrasonic wave irradiation, detects the amount of deformation, and measures intraocular pressure. In Patent Reference 4, a system for measuring intraocular pressure is disclosed. This system can detect the natural vibration of the eyeball by irradiating strong ultrasonic waves from a parametric speaker to the eyeball to vibrate the eyeball and by modulating the frequency of the irradiated ultrasonic waves. Patent Reference 5 discloses a system that detects reflected waves from the eyeball surface of ultrasonic waves irradiated to the eyeball and determines intraocular pressure based on the amount of phase shift of the reflected waves with respect to the irradiation waves.

### Citation List

### Patent Reference

[Patent Reference 1] US patent publication No. 7,909,765
[Patent Reference 2] Publication of Japanese Patent No. 5314090
[Patent Reference 3] Japanese Unexamined Patent Application Publication No. 2020-5679
[Patent Reference 4] Publication of Japanese Patent No. 6289040
[Patent Reference 5] Publication of Japanese Patent No. 5505684
[Patent Reference 6] Japanese Unexamined Patent Application Publication No. S61-8592
[Patent Reference 7] Japanese Unexamined Patent Application Publication No. H03-60629
[Patent Reference 8] Japanese Patent Application Publication No. H08-507463
[Patent Reference 9] Japanese Unexamined Patent Application Publication No. 2000-60801
[Patent Reference 10] Japanese Unexamined Patent Application No. H06-59272
[Patent Reference 11] Japanese Unexamined Patent Application Publication No. 2011-50445
[Patent Reference 12] Japanese Unexamined Patent Application Publication No. 2012-5835

**Non-Patent Reference**

[Non-Patent Reference 1] Direct Experimental Observation of the Crossover from Capillary to Elastic Surface Wave on Soft Gels, October 1998 Physical Review Letters, Volume 81 Number 15, Francisco Monroy and Dominique Langevin
[Non-Patent Reference 2] Surface-wave modes on soft gels, The Journal of the Acoustical Society of America, December r 1998, Y. Onodera and P. K Choi
[Non-Patent Reference 3] Investigating soft materials using surface waves, Journal, the Acoustical Society of Japan, Vol. 56, No. 6 (2000), pp. 445-450, Choi Bokun
[Non-Patent Reference 4] Optical coherence elastography assessment of corneal viscoelasticity with a modified Rayleigh-Lamb wave model, Journal of the Mechanical Behavior of Biomedical Materials, 66 (2017) 87-94, Zhaolong Han et al. US-A-2003/078486 and US-A-5148807 disclose an ocular physical property measuring device according to prior art.

Document US-A-2018 193 194 discloses an intraocular pressure measurement arrangement for measuring pressure of an eye of a patient. At least one source can produce acoustic, nonlinear acoustic, mechanical or a nonlinear mechanical wave from a distance, coupling to the eye to generate at least one surface wave. Upon triggering data acquisition, at least one surface wave from a distance from the eye can be detected to extract surface wave information with pressure information of the eye being based on the surface wave information.

**BRIEF SUMMARY OF THE INVENTION**

**Problems that Invention is to Solve**

However, in the systems such as those disclosed in Patent Reference 1 and Patent Reference 2, the cornea is deformed and measured using an air puff. Therefore, any discomfort to the patient caused by the sound and air being blown when the air puff is ejected is inevitable. In addition, the air puff could scatter adherents on the ocular surface, such as tear fluid. This causes a risk of becoming a source of infection to the surroundings.

Furthermore, in the case of the above-described ultrasonic vibration type, deformation and vibration of the cornea are detected, so the ultrasonic output required to excite eyeball vibration is large. Therefore, in the embodiment of Patent Reference 3, a powerful Langevin type vibrator is used as an ultrasonic oscillator for excitation to increase the sound pressure of the ultrasonic waves. In Patent Reference 4, it is necessary to obtain strong ultrasonic power using a parametric speaker with a very large number of ultrasonic transducer arrays, and in Patent Reference 5, it is necessary to efficiently irradiate ultrasonic waves with acoustic lens. In order to obtain strong ultrasonic power, the ultrasonic oscillator for excitation becomes large and requires a relatively large installation space in front of and near the eye to be examined. In any of the examples of Patent References 3, 4, and 5, there are many constraints on the realization of the device, such as a compatibility with an alignment detection mechanism for the eye to be examined during measurement and a photographing device for photographing eyeball images.

In addition, in terms of these non-contact type tonometers using ultrasonic excitation, the focal position of the excitation ultrasonic irradiation onto the eyeball and the detection position of the eyeball vibration or corneal deformation amount approximately coincide on the eyeball. These methods measure the magnitude of the displacement or amplitude caused by deformation or vibration of the eyeball or the amount of phase shift of the reflected wave of the excited ultrasonic wave by eyeball vibration. Therefore, when the excitation ultrasonic wave irradiation position and the vibration or displacement detection position are at the same position, detection sensitivity is maximized and detection accuracy is optimized.

Therefore, in order to substantially match the focal position of the excitation ultrasonic irradiation with the detection position of the detection device, it is desirable to arrange the irradiation axis of the excitation ultrasonic wave and the detection axis to be coaxial. Alternatively, if coaxiality is not possible, the excitation efficiency deteriorates because the excitation ultrasonic waves are incident obliquely to the eyeball surface. Therefore, it is necessary to compensate for the decrease in measurement sensitivity by increasing the output of the excitation ultrasonic waves even more strongly.

In addition, since the focal position of the ultrasonic irradiation approximately matches the detection position substantially, misalignment of the device against the eye to be examined and deviation in fixation of the eye to be examined will lead to mismatch between the excitation ultrasonic irradiation position and the detection position so that measurement values tend to vary. Because the ultrasonic wave for excitation is not irradiated to an appropriate position on the eyeball, the amplitude of the eyeball vibration and the displacement of the eyeball deformation become small and unstable, and the detection signal also decreases and becomes unstable. As a result, measurements become unstable, such as variations in measured values, and highly reliable measured values cannot be obtained.

Furthermore, in Patent Reference 3 and Patent Reference 4, intraocular pressure is detected based on the magnitude of displacement and the amplitude of the resonance point. Due to this, if a device to detect vibration or displacement is not properly aligned, the detection sensitivity will decrease and a stable detection signal will not be obtained. As a result, it would be impossible to obtain accurate amplitude or displacement. This becomes a fatal factor when measuring amplitude or displacement. Also, this results in reducing the reliability of the measurement.

The present invention has been made in view of the above-mentioned problems and has an objective to provide a non-contact type ocular physical property measuring device that can measure intraocular pressure and physical properties of material mechanical properties of eyeball surface tissue (Young's modulus, shear modulus, viscosity, etc. of eyeball tissue) using surface waves generated at a predetermined point on the eyeball surface without any contact. The present invention is not based on vibrations or displacements of the eyeball or cornea themselves, or changes in phase due to vibrations, as mentioned in the prior art.

**Means to solve the Problems**

In order to solve the above-mentioned problems, the present invention is a non-contact type ocular physical property measuring device according to claim 1 or claim 2.

In this non-contact type ocular physical property measuring device, preferably, further comprises: a modulation means that amplitude-modulates the aerial ultrasonic wave using a modulation frequency of 200 Hz or more and 100 KHz or less, which is lower than the fundamental frequency of the aerial ultrasonic wave.

In this non-contact type ocular physical property measuring device, preferably, wherein the excitation means generates generate a surface tension wave or a leaky surface tension wave, whose frequency ranges from 20 KHz to 50 KHz in a case when being amplitude-modulated by the modulation means, as a surface wave; and wherein the ocular physical property calculation means calculates a surface tension, which is the property, based on the phase velocity of the surface tension wave or the leaky surface tension wave, a curvature of the cornea of the eye to be examined measured by an integrated or separate measuring device, and the below (Equation 1) or (Equation 2), and then calculates an intraocular pressure based on the below (Equation 3).

$$(\text{Equation 1})$$

$$c_c = \sqrt[3]{\frac{\gamma \omega}{\rho}}$$

Herein, $c_c$: phase velocity of surface tension wave, $\gamma$: surface tension, $\rho$: density, $\omega$: angular frequency of surface wave

$$(\text{Equation 2})$$

$$c_{lc} = \sqrt{\frac{\gamma}{\rho}k + 4\frac{\mu}{\rho}}$$

Herein, $C_{lc}$: phase velocity of leaky surface tension wave, $\gamma$: surface tension, $\rho$: density, $\mu$: shear modulus

$$(\text{Equation 3})$$

$$P_i - P_o = \frac{2\gamma}{R}$$

Herein, $P_i$: internal pressure (intraocular pressure), $P_o$: external pressure (atmospheric pressure), $\gamma$: surface tension, R: surface curvature (corneal curvature)

In this non-contact type ocular physical property measuring device, preferably, wherein the excitation means generates Rayleigh wave or Rayleigh-Lamb wave, as a surface wave, whose frequency ranges from 200 Hz to 5KHz in a case when being amplitude-modulate by the modulation means, wherein the ocular physical property calculation means calculates shear modulus, Young's modulus, or Poisson's ratio, which is the property, based on a phase velocity of the Rayleigh wave or the Rayleigh-Lamb wave, and the below (Equation 4) to (Equation 6).

$$\text{(Equation 4)}$$

$$\mu = \frac{E}{2(1+v)}$$

$$E = 2\mu(1+v)$$

Herein, $\mu$: shear modulus, E: Young's modulus, $v$: Poisson's ratio

$$\text{(Equation 5)}$$

$$c_r = \frac{0.87 + 1.12v}{1+v}\sqrt{\frac{\mu}{\rho}}$$

Herein, Cr: Rayleigh wave phase velocity, $\mu$: shear modulus, $\rho$: density, $v$: Poisson's ratio

$$\text{(Equation 6)}$$

$$M = \begin{bmatrix} (k^2+\beta^2)\sinh(\alpha d) & 2k\beta\sinh(\beta d) & (k^2+\beta^2)\cosh(\alpha d) & 2k\beta\cosh(\beta d) & 0 \\ 2k\alpha\cosh(\alpha d) & (k^2+\beta^2)\cosh(\beta d) & 2k\alpha\sinh(\alpha d) & (k^2+\beta^2)\sinh(\beta d) & 0 \\ -(k^2+\beta^2)\sinh(\alpha d) & -2k\beta\sinh(\beta d) & (k^2+\beta^2)\cosh(\alpha d) & 2k\beta\cosh(\beta d) & \frac{\rho_F\omega^2}{\mu^*} \\ 2k\alpha\cosh(\alpha d) & (k^2+\beta^2)\cosh(\beta d) & -2k\alpha\sinh(\alpha d) & -(k^2+\beta^2)\sinh(\beta d) & 0 \\ \alpha\cosh(\alpha d) & k\cosh(\beta d) & -\alpha\sinh(\alpha d) & -k\sinh(\beta d) & -\alpha_F \end{bmatrix}$$

$$\mu^* = \mu + i\omega\eta$$

$$\alpha_F^2 = k^2 - \frac{\omega^2}{c_F^2}$$

$$k = \frac{\omega}{c_p}$$

Herein, Cp: phase velocity of Rayleigh-Lamb wave, $\rho$: corneal density, $\rho$F: water density d: corneal thickness x 1/2, k: wave number of surface wave, $\omega$: angular frequency of surface wave, $\mu$: shear elasticity rate, $\eta$: viscosity coefficient,

cF: sound velocity of water

In this non-contact type ocular physical property measuring device, preferably, wherein the detection means detects a surface wave using one of an ultrasonic reflection method, an optical triangulation method, a confocal method of optical coaxial light beams having multiple wavelengths, a Fourier domain optical interferometer using an optical heterodyne method, or a vibration detection by a laser Doppler interferometer.

In this non-contact type ocular physical property measuring device, preferably, wherein the excitation means and the detection means are arranged such that an intersection of an irradiation axis of the excitation means and a detection axis of the detection means coincides with the center of corneal curvature.

In this non-contact type ocular physical property measuring device, preferably, further comprises a corneal curvature measurement function that measures a corneal anterior curvature along the point from an excitation point by the excitation means and a detection point by the detection means.

In this non-contact type ocular physical property measuring device, preferably, further comprises a measurement function that measures a corneal curvature and a corneal thickness along the point from an excitation point by the excitation means and a detection point by the detection means using a light-section method.

In order to solve the above-mentioned problems, the present invention is a non-contact type ocular physical property measuring method according to claim 8 or claim 9.

**Effects of the Invention**

The non-contact type ocular physical property measuring device of this invention comprises: an excitation part that excites at least one or more excitation points on an eyeball by using an irradiation wave to generate a surface wave on a surface of an eye to be examined; a detection part that detects the surface wave generated by the excitation part at at least one or more detection points, which are different from the excitation points, on the eyeball; a surface wave processing part that analyzes the surface wave detected by the detection part; and an ocular physical property calculation part that calculates physical properties of the eyeball based on the analyze result by the surface wave processing part. With this configuration, the present invention can measure an intraocular pressure using surface waves on the eyeball intentionally generated by the excitation part without any vibrations of the eyeball itself or the cornea itself. Due to this, the examinee never feel any discomfort while measuring an intraocular pressure and so on. In addition, it is possible to achieve a stable measurement without being affected by the amplitude of the waveform. Furthermore, since the excitation point and detection point are separated, the observation optical system and alignment optical system can be easily arranged.

**Brief description of Drawings**

FIG. 1 is a diagram showing the basic configuration of a surface wave excitation part and a detection part for the cornea of an eye to be examined, wherein those parts are included in an ocular physical property measuring device according to the present embodiment.

FIG. 2 is a functional block diagram of the ocular physical property measuring device.

FIG. 3 is a diagram showing an example of a waveform obtained by amplitude-modulating an ultrasonic wave for excitation of a surface wave using a modulator, which is included in the ocular physical property measuring device.

FIG. 4 is a diagram showing the generation of low-frequency sound pressure using a parametric speaker system, in a case when a transducer comprises a plurality of ultrasonic transducers for excitation of surface waves, which are provided in the ocular physical property measuring device.

FIG. 5 is an explanatory diagram when a surface wave is generated on the surface of the eye to be examined by the photoacoustic effect of pulsed light in the excitation part included in the ocular physical property measuring device.

FIG. 6 is a diagram showing an example of a generation pattern of modulated pulsed light for generating a surface wave by pulsed light using the modulator.

FIG. 7 is a diagram showing an example of a case where an external modulation method of laser pulsed light is used in the modulator.

FIG. 8 is a diagram showing an example of a case where a laser diode direct modulation method is used in the modulator.

FIG. 9 is a diagram showing an example of surface wave detection using an ultrasonic reflection method in the detection part, which is included in the ocular physical property measuring device.

FIG. 10 is a diagram showing an example of surface wave detection using an optical triangulation method in the detection part.

FIG. 11 is a diagram showing an example of surface wave detection using a confocal method of optical coaxial light beams having multiple wavelengths in the detection part.

FIG. 12 is a diagram showing an example of surface wave detection by a Fourier domain optical interferometer using an optical heterodyne method in the detection part.

FIG. 13 is a diagram showing an example of surface wave detection using a surface wave detection by a laser Doppler interferometer using an optical heterodyne method in the detection part.

FIG. 14 is a diagram showing an example of the arrangement of a surface wave excitation part and a detection part, which are included in the ocular physical property measuring device.

FIG. 15 is a diagram showing an example of a corneal curvature measurement function, which is attached to the ocular physical property measuring device.

FIG. 16 is a diagram showing an example of the relationship between the imaging state of a punctate target for measuring the corneal curvature of the eye to be examined and the curvature line to be measured in the ocular physical property measuring device.

FIG. 17 is a diagram showing an example of measuring the corneal thickness and corneal curvature of the eye to be examined using a light-section method in the ocular physical property measuring device.

FIG. 18 is a diagram showing an example of the arrangement of a corneal curvature and corneal thickness measuring system using a light-section method and an intraocular pressure measuring system using a surface wave in the ocular physical property measuring device.

FIG. 19 is a flowchart showing an example of the operation steps in terms of the ocular physical property measuring device.

**Description of Embodiments**

(Embodiment)

Herein, embodiments of the present invention will be described in the following order.

(1) Basic configuration of the measurement method
(2) Surface waves generated on the ocular surface
(3) Configuration of excitation means for generating surface waves
(4) Configuration of detection means for detecting surface waves
(5) Arrangement of excitation means and detection means
(6) Processing of obtained data
(7) Embodiment of corneal anterior curvature measurement function
(8) Embodiment of corneal curvature and corneal thickness measurement using light-section method

(1) Basic configuration of the measurement method

The present invention utilizes the ocular surface waves of the eye to be examined. Based on the phase velocity thereof, this invention determines the intraocular pressure of the eye to be examined and the material properties of the tissue of the eye to be examined.

Therefore, in a case when the excitation point and the detection point are each located at one point, it is impossible to measure the propagation of the surface wave unless the detection point is measured at a point different from the excitation point. However, this does not apply when there are multiple excitation points or detection points.

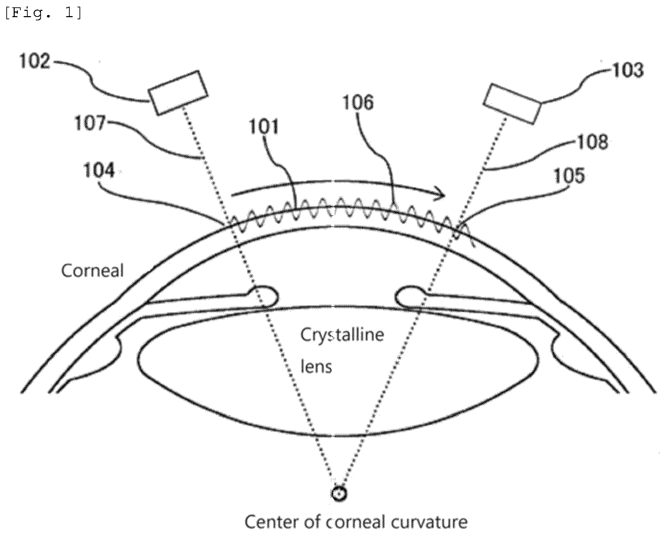

In terms of the non-contact type ocular physical property measuring device according to the present embodiment, FIG. 1 shows the simplest configuration that has one excitation point 104 and one detection point 105. The excitation part 102 is arranged against the ocular surface 101 so that the excitation irradiation axis 107 can be almost coaxial with the normal line of the excitation point 104. The detection part 103 is arranged against the ocular surface 101 so that the detection axis 108 can be almost coaxial with the normal line of the detection point 105. The intersection point on the extended line of the excitation irradiation axis 107 and the detection axis 108 substantially coincides with the center of corneal curvature. The ultrasonic waves or pulsed light (It is preferable to use continuous pulsed light from a coherent light or non-coherent light source.) irradiated onto the ocular surface by the excitation unit 10, which is arranged in the above-mentioned manner, excites a surface wave 106 having an amplitude against the ocular surface vertically. The surface waves 106 propagate from the excitation point on the ocular surface with a certain frequency and phase velocity determined by the excitation means just like small waves generated on the water surface when a stone is thrown onto the water surface.

Therefore, it is possible to determine the frequency and phase velocity of the surface waves by detecting the waves at a predetermined point.

Herein, surface waves will be explained. Waves that propagate in or on soft tissue include body waves that propagate in the tissue, and surface waves and guided waves that propagate on the surface of the tissue. Herein, surface waves or guided waves are collectively referred to as the propagation of waves occurring on the tissue surface.

Body waves include longitudinal elastic waves and transverse shear elastic waves. Even in biological tissues, they are applied to image diagnostic devices using ultrasonic wave as longitudinal elastic waves, and ultrasonic wave elastography to diagnose tissue hardness by measuring shear elastic waves of transverse waves.

As for surface waves, there are Rayleigh waves as surface elastic waves that effect restoring force by shear elasticity acts, surface tension waves by the restoring force of surface tension, and leaky surface tension waves, which propagate with the combined effect of shear elasticity and surface tension. Regarding these surface waves, measurement examples such as hardness measurement of gel-like substances, are known.

Furthermore, Lamb waves are known as waves that propagate in plate-shaped media. Unlike Rayleigh waves, which propagate in semi-infinite media, Lamb waves propagate in plate-like media under the condition where the outside of both boundary surfaces of the plate satisfies the mechanically free boundary condition such as air or a vacuum state.

However, ocular tissues such as cornea, conjunctiva, and sclera have a layered structure. For example, the outer surface of cornea is in contact with air, and the inner boundary surface of cornea is a thin layer being in contact with anterior aqueous humor. The boundary conditions for each interface are different, and it is necessary to consider the case where one of the interfaces is in contact with a liquid substance. In such cases, the Rayleigh-Lam model is known as a guided wave concerning the boundary conditions with another material that is in contact with the material at the boundary.

The shear modulus G and viscosity $\eta$ of the medium can be calculated by the phase velocity Cr and the density $\rho$ of the medium using the Rayleigh waves and Rayleigh-Lamb waves. Further, the phase velocity Cc and the density $\rho$ can be determined from the surface tension wave, and the surface tension $\gamma$ of the medium can be determined from the angular frequency $\omega$. Furthermore, the phase velocity Cl and the shear modulus G can be determined from the leaky surface tension wave, and the surface tension $\gamma$ can be determined from the angular frequency $\omega$.

As mentioned above, it is possible to determine the shear modulus G and viscosity $\eta$ by measuring the phase velocity of Rayleigh-Lamb waves relating to the tissue surface. Surface tension $\gamma$ is determined by measuring the phase velocity of surface tension waves or leaky surface tension waves, and internal pressure, that is, intraocular pressure in the case of an eyeball, can be determined from the surface curvature radius and surface tension using Laplace's law.

Here, the surface waves that propagate on the surface of the eyeball are not vibrations of the eyeball or cornea themselves, but are waves that are localized and propagated only near the surface of the tissue. The power required to generate this surface waves is smaller than the power required to vibrate the cornea or eyeball itself. Furthermore, since what is detected is the propagation velocity, phase, or frequency spectrum of the surface waves, there is little influence on amplitude changes of the surface waves, and it is possible to realize a stable measurement against various external influences in clinical practice.

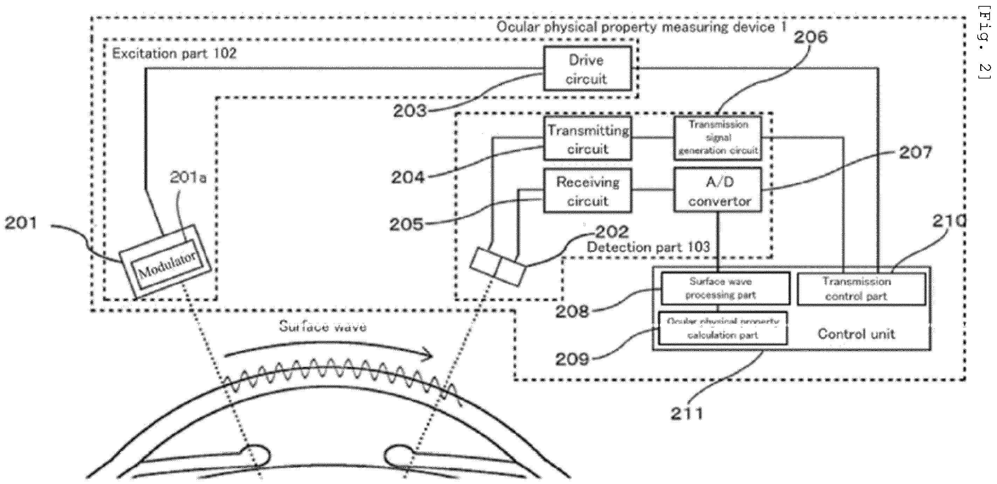

Next, the functional configuration of the non-contact type ocular physical property measuring device 1 according to the present embodiment will be described by referring to FIG. 2. FIG. 2 shows an example of the ocular physical property measuring device 1. An excitation unit 201 that excites the eyeball surface generates a surface wave by exciting the eyeball surface, and a detection unit 202 detects the surface wave. The excitation unit 201 is driven by a drive circuit 203, and the drive circuit 203 is controlled by a transmission control part 210 of a control unit 211. The excitation unit 201 comprises a modulator 201a for amplitude-modulating an ultrasonic wave or pulsed light using a modulation frequency, which is lower than the oscillation frequency of the ultrasonic wave or pulsed light. The detection unit 202 has a transmitting part and a receiving part integrated therewith. The transmitting part driven by the transmitting circuit 204 for detection sends a transmission signal for detection to the ocular surface, and the receiving part receives the signal reflected from the ocular surface and sends the detection signal to the receiving circuit 205 for detection. In the receiving circuit 205 for detection, for example, an amplified received wave is converted into a digital signal by an A/D converter 207 and send the digital signal to the control unit 211. A surface wave processing part 208 in the control unit 211 processes the received waves, identifies components of the surface waves among the received waves, and measures the phase and delay time of the surface waves. An ocular physical property calculation part 209 calculates the phase velocity based on the phase and delay time of the surface waves, and calculates the intraocular pressure or the material properties of the eye tissue.

As the excitation unit 201 that excites the surface waves, there is an excitation method using ultrasonic waves and an excitation method using pulsed light. As for the ultrasonic method, the ocular surface is vibrated by the sound pressure of the ultrasonic waves irradiated to a predetermined position on the ocular surface so as to generate surface waves. As for the method using optical energy, short pulsed light is irradiated onto the ocular surface, and the tissue at the focal point of the irradiated pulsed light absorbs the light energy. This causes its temperature to rise and instantaneous thermal expansion. As a result, ultrasonic waves can be generated within the tissue. The generated ultrasonic waves propagate on the tissue surface and excite surface waves.

As mentioned below, methods for detecting surface waves include a detection method using an ultrasonic reflection method, a minute displacement detection method using optical triangulation, a multi-wavelength coaxial confocal method, and an optical heterodyne method. The ultrasonic reflection method uses ultrasonic waves with a frequency several times higher than the frequency of the generated surface waves. The ultrasonic waves are sent to and received from the surface wave detection point. The surface waves on the ocular surface can be detected by demodulating the reflected waves. In optical triangulation, a light beam is irradiated onto a detection point, and the light reflected from the detection point is detected by a plurality of light receiving elements or a one-dimensional or two-dimensional imaging device. Due to this, surface waves are detected from changes in the output of the light-receiving element and changes in the bright spot position of the image sensor. Those changes are caused from periodic changes in the reflection angle due to surface vibration. In the differential detection method of multiple wavelength light beams, multiple light beams with different wavelengths are coaxially irradiated onto the ocular surface, and the optical design is such that the focal position of each wavelength light beam is slightly shifted. When alignment is performed so that the corneal surface is within the focal position region, the intensity of reflected light of each wavelength fluctuates due to vibration of the ocular surface. As a result, the reflected light intensity of each wavelength is differentially amplified to detect surface waves. The optical heterodyne methods include a method that measures phase changes due to minute fluctuations on the ocular surface detected by a Fourier domain optical interferometer, and a method that detects surface waves by detecting minute vibrations on the ocular surface using a laser Doppler vibrometer.

To detect the phase velocity of the surface wave, firstly, the phase difference between the detected surface wave signal and the drive signal of the excitation unit drive circuit 203 is measured. Then the phase velocity can be calculated from the phase difference and the distance from the excitation point to the detection point. If there are multiple detection points, the phase of the surface wave at each detection point can be detected, and the phase velocity can be calculated based on the phase difference and the distance between the detection points.

Once the phase velocity of the Rayleigh wave or Rayleigh-Lamb wave is determined, the shear modulus, Young's modulus, and viscosity of the tissue can be calculated using the equations described below.

Once the phase velocity of the surface tension wave or leakage surface tension wave on the corneal surface is determined, the tension on the corneal surface can be determined from the equation described below. Then internal pressure, that is intraocular pressure, can be calculated from the average curvature of the ocular surface and surface tension using Laplace's law.

(2) Surface waves generated on the ocular surface

Among elastic waves, which are surface waves generated on the surface of living tissues, there are Rayleigh waves and Rayleigh-Lamb waves in which shear elasticity and viscosity act as restoring forces in the propagation process, surface tension waves in which surface tension acts as restoring forces, and leakage surface tension waves that act as a restoring force that is a mixture of shear elasticity, viscosity, and surface tension. For example, in the case of Rayleigh-Lamb waves generated on the corneal surface, the zero-order of the asymmetric mode can be observed. The phase velocity of the zero-order is relatively slow, about 1 to 5 m/sec, and there is frequency dispersion, so the phase velocity differs depending on the frequency. It is possible to detect frequencies from about 200 Hz to 5KHz, and when the frequency is outside this range, the attenuation increases and detection becomes difficult. Since this measurable frequency varies depending on the Young's modulus of the eye tissue and the thickness of the tissue layer, it is necessary to measure with multiple frequencies.

In the case of surface tension waves and leaky surface tension waves, in order for surface tension to become dominant as a restoring force, the wavelength must be short and the waves must be localized to the biological surface. In this case, the frequency becomes high and the detection can be realized with higher frequency, compared to Rayleigh waves and Rayleigh-Lamb waves. The frequency of surface tension waves has a shorter wave length, compared to the thickness of the cornea, and can be detected at a frequency of 20 KHz or higher (for example, 20 to 50 KHz), which is less affected by Rayleigh-Lamb waves.

(3) Configuration of excitation means for generating surface waves

As for the surface wave excitation means, there are excitation means using ultrasonic waves and excitation means using optical energy. In the case of aerial ultrasonic wave, the frequency can range from about 20 KHz to about 1 MHz, but in order to obtain effective power for excitation, it is appropriate to range from about 20 KHz to about 100 KHz. In the case of an aerial ultrasonic transducer with 100 KHz or more, not only the radiated sound pressure is low, but also the attenuation due to air propagation is large. When ultrasonic waves propagate through the air, the attenuation coefficient is $1 \times 10^{-11} \times f^2$ (m$^{-1}$), where f is the frequency of ultrasonic waves, and is proportional to the square of the frequency. The attenuation coefficient is 0.1(m$^{-1}$) at 100KHz and the attenuation increases exponentially.

The aerial ultrasonic transducers, which are used generally as ultrasonic sensors, may not have enough sound pressure with one transducer. In such cases, sufficient ultrasonic output can be obtained by driving multiple ultrasonic transducers. Furthermore, the phase of the ultrasonic waves is controlled by controlling the phase of each drive signal from a plurality of ultrasonic transducers. And then, the focal point can be controlled by configuring a phased array transducer that controls the focal point on the eye surface freely. Even when a plurality of ultrasonic transducers are used, the sound pressure of the ultrasonic waves is not as high as that of vibration detection using corneal vibration. Therefore, the number of transducers when configuring an ultrasonic transducer array is not large and is limited.

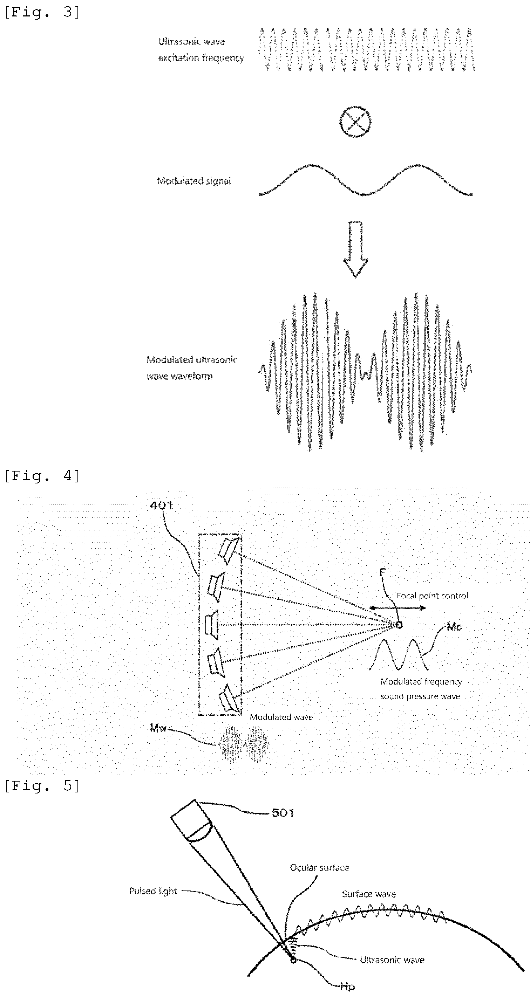

Furthermore, in order to excite Rayleigh waves or Rayleigh-Lamb waves on the ocular surface, the ocular surface must be locally excited by a frequency with the audible range, wherein said frequency is lower than that of ultrasonic wave. Therefore, by amplitude-modulating a high-frequency ultrasonic wave with a low frequency, it is possible to generate a sound pressure wave, which has the same low frequency as the amplitude-modulation frequency near the focal point. FIG. 3 shows how the modulator 201a is used to superimpose a modulation signal of a low frequency on the oscillation frequency of an ultrasonic transducer in order to perform amplitude-modulation and generate amplitude-modulated ultrasonic waves. Furthermore, by performing phased array control and frequency amplitude-modulation using multiple ultrasonic wave transducers, low-frequency sound waves are generated only near the focal point while maintaining sharp directivity. As a result, a predetermined point on the ocular surface can be excited by an amplitude-modulation frequency selectively. This is the well-known principle of parametric speakers. FIG. 4 shows the generation of low frequency sound pressure near the focal point of the ultrasound beam with the parametric speaker configuration. For example, in FIG. 4, a modulated ultrasonic wave Mw is transmitted from a transducer array 401 comprising a plurality of ultrasonic transducers at the excitation unit 201. And FIG. 4 shows that the ultrasonic waves are focused at the focal point F, and a sound wave Mc having a modulated frequency component with a low frequency, is locally generated. It is also possible to control the focal point by changing the drive signal phase of each individual transducer in the transducer array 401 to control the focal point back and forth.

When the excitation means is optical energy, excitation using short pulsed light is possible. FIG. 5 shows the excitation of surface waves by pulsed light. The light source 501 irradiates the ocular surface with continuous pulses of pulsed light, and the irradiated pulsed light energy is absorbed by molecules near the heat point Hp located at the focal point. As a result, ultrasonic waves are generated in the intraocular tissue just below the ocular surface by the instantaneous thermal expansion and contraction of the tissue. The ultrasonic waves reach the surface and excite surface waves. A method of generating ultrasonic waves in a living body using light is known as the photoacoustic effect, and an image diagnostic apparatus using photoacoustic imaging using this principle is known.

The pulsed light used for the photoacoustic effect is very short, because the pulsed light applies the order of nanoseconds. The frequency of ultrasonic waves generated in a living body as a photoacoustic effect with pulsed light is approximately 1 MHz to 10 MHz. Therefore, ultrasonic waves generated by simple continuous pulses cannot excite surface waves having low frequencies in the order of KHz. In order to solve this problem, the pulsed light uses continuous pulses with 100 KHz or more. In addition, the intensity is modulated at a frequency that is as low as the frequency of the surface wave, and that generates the continuous pulsed light.

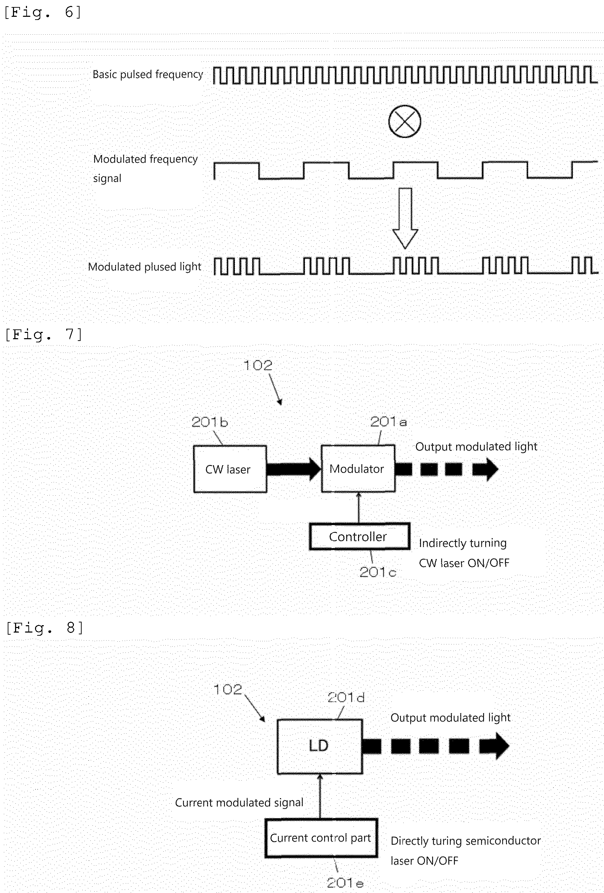

This superimposes sound pressure changes with low frequencies on surface waves and excites the surface waves. FIG. 6 shows that continuous pulsed light having a basic pulsed frequency is modulated by a modulated frequency signal to produce modulated pulsed light. In other words, it is necessary to continuously irradiate amplitude-modulated continuous pulsed light in which the power intensity of the optical pulse is amplitude-modulated by a modulation means, or to irradiate a burst wave of 10 cycles or more of this amplitude-modulated continuous pulsed light.

As a light intensity modulation method, FIG. 7 shows an external direct modulation method in which a CW laser 201b provided in the excitation unit 201 is directly turned ON/OFF by the modulator 201a that receives an electrical adjustment signal from the controller 201c. FIG. 8 shows a direct modulation method in which the driving current of a semiconductor light source LD (semiconductor laser, SLD, light emitting diode, etc.) 201d is modulated by a current control part 201e in the excitation unit 201.

Light sources for pulsed light are such as light emitting diodes, semiconductor lasers, fiber lasers, and super luminescent diodes. The wavelength of the light source is preferably a wavelength at which the optical absorption rate of the ocular surface tissue is high. The cornea is a transparent tissue, and its light absorption rate is low in the region between 380 nm and 1400 nm, and the absorption rate is high at other wavelengths, so ultraviolet and near-infrared rays can be the candidates. However, when considering biosafety, the wavelength is preferably from the near-infrared region around 1400 nm to the infrared region around 3000 nm, since the ultraviolet region is highly invasive to the living body and the power of light cannot be increased. Opaque tissues other than the cornea have a high absorption rate of light from the visible light region of 400 nm or more to the near-infrared region. In particular, the absorption rate of blood hemoglobin increases rapidly at wavelengths of 600 nm or less. Therefore, efficient excitation can be achieved by using pulsed light in the range from 400 nm to 600 nm when the capillaries on the ocular surface are regarded as heat points. When using a surface tissue other than the cornea, such as the conjunctiva, as an excitation point, pulse light is irradiated to the capillaries of the conjunctiva as a heat point Hp, and surface waves are excited on the conjunctival surface, and the surface waves propagated to the cornea are detected on the cornea. This enables efficient measurement.

As for the excitation method for generating surface waves, excitation using continuous waves was mainly explained. However, excitation using burst waves is also possible. However, in the case of a burst wave, in order to stabilize the output of the excitation means and further stabilize the amplitude of the surface wave, it is necessary to output continuous waves having a certain constant wave number. For example, this is because a certain amount of time is required for the sound pressure of the ultrasonic transducer to reach its peak in terms of excitation by ultrasonic waves. In addition, in terms of excitation by pulsed light, it takes a lot of time for the surface waves to rise. Therefore, in the case of surface wave excitation using burst waves or burst pulses, ultrasonic waves, modulated ultrasonic waves, or modulated pulsed light are transmitted at a transmission time such that the number of detectable surface waves is more than 10 waves (10 cycles or more). The transmission time of the burst wave depends on the characteristics of the ultrasonic transducer, pulsed light source, etc., and is therefore determined according to these characteristics.

(4) Configuration of detection means for detecting surface waves

The detection means for surface wave detection include an ultrasonic reflection method, an optical triangulation method, an optical multi-wavelength coaxial confocal method, and an optical heterodyne method.

When the detection means is an ultrasonic reflection method, detection is performed using continuous ultrasonic waves or burst ultrasonic waves having 100 or more waves. After the ultrasonic waves transmitted from the transmitting ultrasonic transducer reach the ocular surface, the waves are reflected and scattered, and then returned. When the ultrasound waves reflect on the ocular surface, they are modulated by surface wave vibrations and return to the receiving ultrasound transducer with the surface wave vibration components superimposed with it. By demodulating and detecting this reflected wave, it is possible to demodulate the vibration frequency component modulated by the surface wave vibration on the eye surface. It is possible to calculate the phase and frequency of the surface wave by measuring the frequency and phase of this demodulated wave.

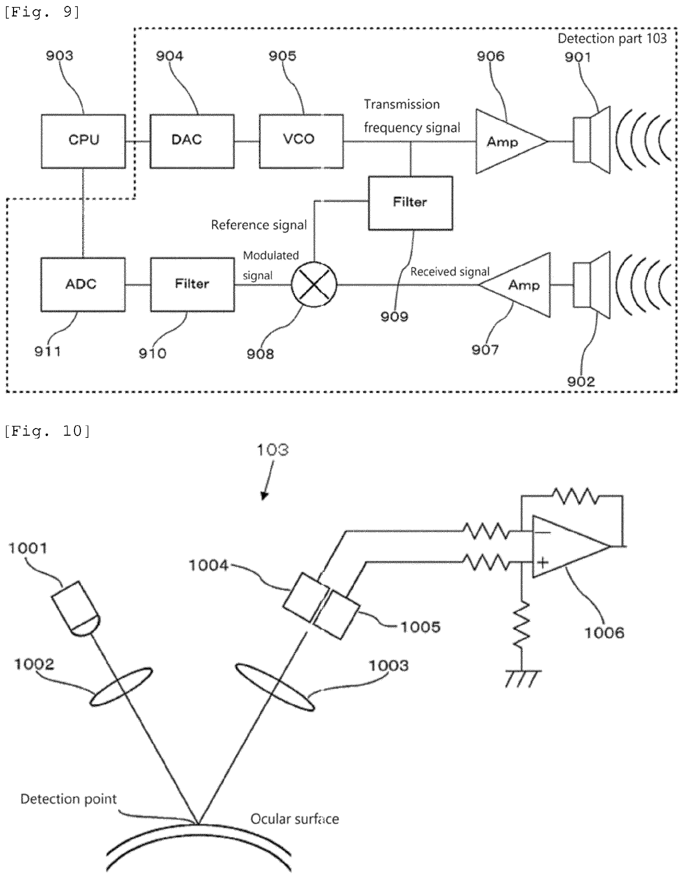

FIG. 9 shows one embodiment of surface wave detection using ultrasonic waves. In this embodiment, a transmitting ultrasonic transducer 901 and a receiving ultrasonic transducer 902 are provided separately. By using the transmitting and the receiving ultrasonic transducers separately, it is possible to use continuous ultrasonic waves for detection. Therefore, there is a merit that surface waves can always be detected. A digital value for controlling the oscillation frequency of the voltage variable oscillator 905 is output by the control CPU 903 to the D/A converter 904, and a control voltage corresponding to the digital value is output by the D/A converter 904 to the voltage variable oscillator 905. The voltage variable oscillator oscillates at a frequency corresponding to the voltage. The oscillated signal is amplified by a transmission amplifier 906. Then, the ultrasound for detection is transmitted to the ocular surface by driving the transmission ultrasound transducer 901. The ultrasonic waves reflected and returned from the ocular surface are modulated with the frequency of the surface wave due to the surface wave vibrations on the ocular surface. The receiving ultrasonic transducer 902 receives the returned ultrasonic waves, and the received signal amplified by a receiving amplifier 907 is input to a multiplier 908. The transmission signal output from the voltage variable oscillator 905 is also used as a reference signal to demodulate the received signal. Therefore, the surface wave component contained in the received signal is demodulated by demodulating the received signal by generating the reference signal filtered by a reference signal band-pass filter 909 that passes only the transmission frequency band, and by multiplying the reference signal with the received signal by the multiplier 908. A demodulated signal band-pass filter 910 removes frequency components other than surface waves, and an A/D converter 911 converts the signal into a digital value, which is input to the control/calculation CPU 903 for calculation processing. In this case, the control/calculation CPU 903 is placed in the control unit 211 shown in FIG. 2, and here, the physical property values of the surface waves such as surface tension and the intraocular pressure of the eyeball are calculated based on these physical property values.

If a burst wave is used for the ultrasonic transmission for detection, the ultrasonic transducer can be used for both transmission and reception, and surface wave vibrations can be detected with one transducer. It is also possible to digitally convert the returned received signal without demodulating or detecting the signal by using an A/D converter. And it is possible to input the digital signal to the calculation CPU, and to calculate the frequency spectrum and phase change of the surface wave component by Fourier transform.

Herein, the frequency of the ultrasonic waves for detection should be at least about 10 times as high as the frequency of the surface waves excited by the excitation means. This is because the fundamental frequency of the ultrasonic wave and the frequency components of the surface wave have to be separated one another to accurately detect the surface wave, when demodulation/detection and Fourier transform are performed. Therefore, the reason why the burst waves for detection are set to 100 waves or more, is to detect and analyze at least 10 waves or more of surface wave components in order to improve the phase detection accuracy of the surface waves. As a result, the ultrasonic wave for detection has 100 waves, which is about 10 times as high as the surface wave frequency.

Next, the case where the detection means for surface wave detection is detected by optical triangulation will be described with reference to FIG. 10. In this case, the detection point is irradiated with a detection light beam, and the light reflected, scattered and returned from the detection point is detected by a plurality of light receiving elements, optical position sensors, optical line sensors, or the like. In the case when the detection point surface vibrates due to surface wave vibration, the angle and intensity distribution of the light returning from the detection point will change, and the imaging position on the light receiving side image formation surface will also change. Therefore, in the case of multiple light-receiving elements, it is possible to detect vibrations by differentially amplifying the output change of each light-receiving element, and in the case of the optical position sensors or optical line sensors, it is possible to detect the change in the imaging position itself. As a result, the change in position can be detected as surface wave vibration. FIG. 10 is an example of detection means using optical triangulation. The light emitted from a surface wave detection light source 1001 reaches the ocular surface via an objective lens 1002, and the light reflected at the detection point enters a light receiving element A1004 and the light receiving element B1005 via an imaging lens 1003. In the case when the detection point vibrates due to surface waves, the optical axis angle of the light reflected at the detection point changes in accordance with the vibration. Therefore, the amount of light entering each of the light receiving element A1004 and the light receiving element B1005, changes relatively. The surface wave vibration at the detection point is detected by differentially amplifying the electric signals output from the light receiving element A1004 and the light receiving element B1005 by using the differential amplifier 1006.

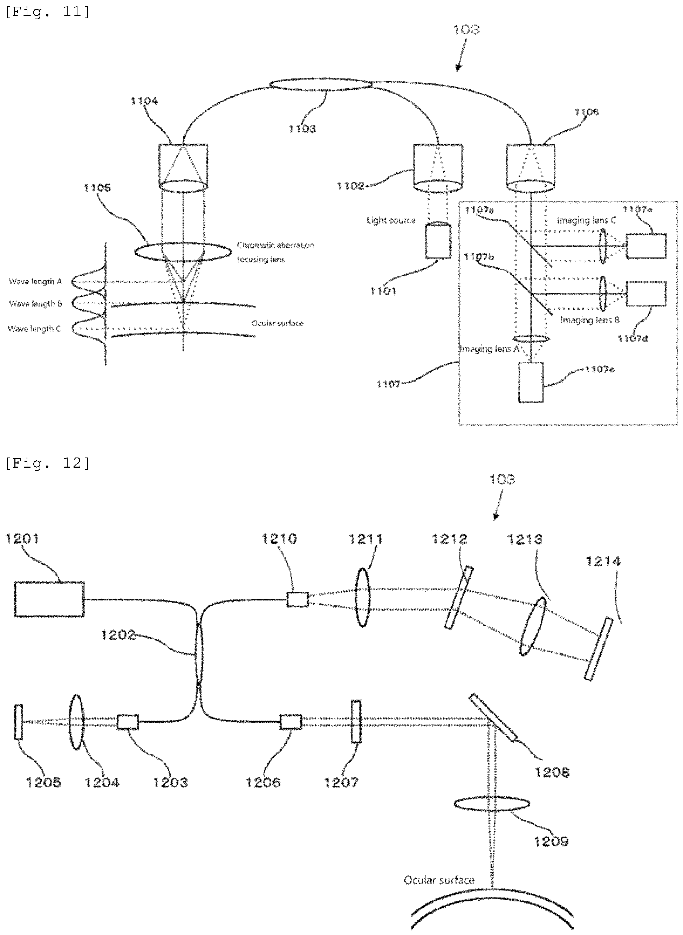

Next, a case when the detection means uses a confocal method of optical coaxial light beams having multiple wavelengths, will be described by referring to FIG. 11. In this case, the measurement light beam is irradiated by a light source having multiple wavelengths or white light, and an optical system has a chromatic aberration focus such that the focal point shifts little by little depending on the wavelength. In the case when minute vibrations due to surface waves occur at the detection point on the ocular surface, the amount of reflected light of each wavelength whose focal points differ slightly changes in accordance with the vibration. In other words, the reflected light of wavelengths close to the focal point is strong, while the reflected light of wavelengths away from the focal point is weak. Furthermore, the confocal optical system allows for sharper reflection peaks near the focal point of each wavelength. Therefore, in the case when the reflected and returned light is separated into multiple wavelengths and detected by a light receiving element, the amount of light entering the light receiving element of each wavelength changes significantly even with minute vibrations. It is possible to detect vibrations at the detection point due to surface waves by differentially amplifying the electrical signals from the light receiving elements of each wavelength.

FIG. 11 shows an embodiment of a multi-wavelength coaxial confocal method. A light beam emitted from a light source 1101 passes through a fiber collimator 1102, a fiber coupler 1103, and a fiber collimator 1104, and is irradiated onto a detection point on the ocular surface by a chromatic aberration focusing lens 1105, with the focus of each wavelength being slightly shifted. In this example, the focus of wavelength A, wavelength B, and wavelength C, is slightly shifted from the detection point, so the detection peak of each wavelength shifts. This shows that when the detection point on the ocular surface shifts, the amount of reflected light of each wavelength changes. The light beam reflected at the detection point enters a spectral detection unit 1107 via a chromatic aberration lens 1105, a fiber collimator 1104, and a fiber collimator 1106. The light beam incident on the spectroscopic detection unit is split into wavelength A, wavelength B, and wavelength C by hot mirror 1107a and hot mirror 1107b. Then these enter a photodetector 1107c, a photodetector 1107d, and a photodetector 1107e via an imaging lens A, which becomes confocal for each wavelength, an imaging lens B, and an imaging lens C. Surface wave vibrations are detected by differentially amplifying the electrical signals output from each photodetector.

Next, a case where the detection means is phase detection with a Fourier domain optical interferometer using an optical heterodyne method will be described by referring to FIG. 12. In this case, a light beam emitted from a low-coherence light source such as a super luminescent diode or a wavelength-swept laser light source, is divided into a light beam that irradiates the measurement target and a light beam that serves as a reference light within the optical interferometer. The light beam irradiated onto the object to be measured is irradiated onto the ocular surface by the interferometer, reflected on the ocular surface, and then enters the optical interferometer again. The light beam incident on the optical interferometer interferes with the reference light inside the interferometer and is detected as a spectral distribution waveform with an interference spectral distribution and a beat signal with a beat frequency. The interference peak and phase of the light rays reflected from the ocular surface and returned are calculated by Fourier transforming these spectral distribution waveforms and beat signals. Microfluctuations caused by surface waves are detected as changes in phase. As a result, the surface waves can be detected.

FIG. 12 shows an embodiment of a Fourier domain interferometer using the spectral domain method. A light beam emitted from a super luminescent diode (SLD) light source has low coherence and a wide wavelength band. This light beam enters a fiber coupler 1202 via an optical fiber and is split into two optical paths. One path is projected onto a reference mirror 1205 via a fiber collimator 1203 and an achromatic lens 1204, is reflected, and returns to a fiber coupler 1202 via an achromatic lens 1204 and a fiber collimator 1203 again. In the other path, the split light beam is sent from the fiber coupler through a fiber collimator 1206 and an aperture 1207, and is waved or set at a predetermined angle by a galvanometer mirror 1108 for scanning. Then the beam is projected onto the ocular surface via an objective lens 1209. The light beam reflected from the ocular surface returns to a fiber coupler 1202 via an objective lens 1209, a galvanometer mirror 1208, an aperture 1207, and a fiber collimator 1206. In the fiber coupler, the reference light and the reflected light returning from the ocular surface are superimposed and become interference light. The interference light is projected onto a CCD 1214 via a fiber connector 1210, a relay lens 1211, a diffraction grating 1212, and an achromatic lens 1213. The CCD 1214 outputs spectral distribution data separated by the diffraction grating 1212. This spectral data is Fourier-transformed by a calculation circuit to calculate the interference peak and phase to detect minute vibrations caused by the surface waves.

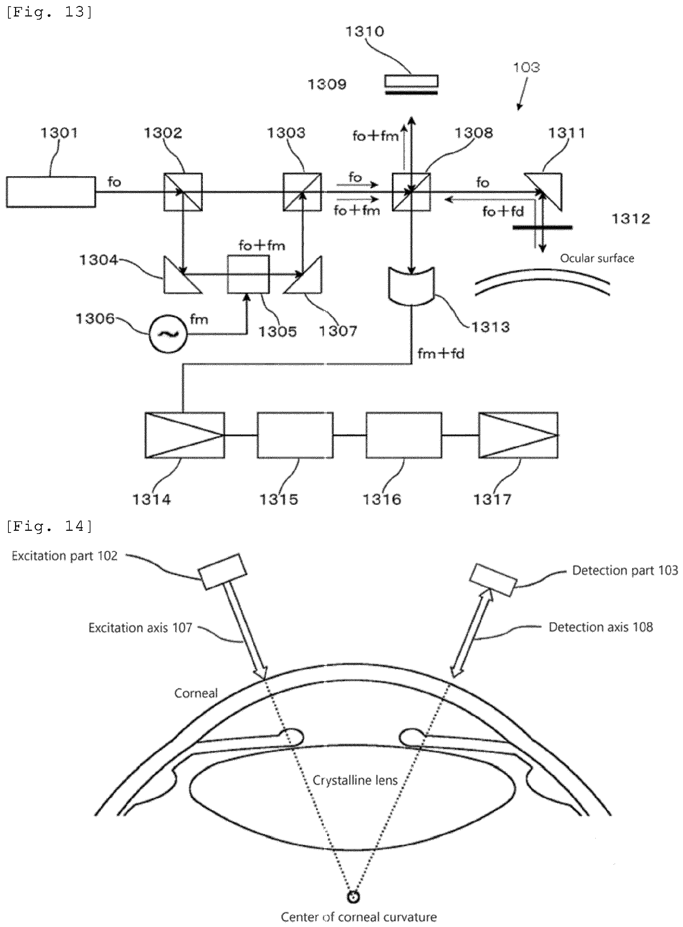

Next, a case where the detection means is a laser Doppler interferometer using optical heterodyne will be described by referring to FIG. 13. In this case, the beam emitted from the laser light source is split into two. One beam is irradiated onto the ocular surface, and the other beam is modulated by an acousto-optic modulator at a constant carrier frequency fm. The light beam reflected on the ocular surface undergoes a Doppler shift with a frequency fd due to surface wave vibration on the ocular surface and returns to the interferometer again. Then it interferes with the light modulated by the acousto-optic modulator to generate a beat frequency of fm±fd. Since fm among this beat frequency is a constant frequency, only the Doppler shift frequency fd changes due to surface wave vibration. Surface wave vibrations can be detected by FM demodulating this frequency change.

FIG. 13 shows an embodiment of the laser Doppler interferometer. A light beam emitted from a laser light source 1301 having an optical frequency fo is split by a polarization beam splitter 1302 into two light beams, which are S-polarized light and P-polarized light. The S-polarized light beam is reflected by a polarizing beam splitter 1302 and enters an acousto-optic modulator (AOM) 1305 via a mirror 1304. The frequency is modulated to fo+fm by an acousto-optic modulator, reflected by a polarizing beam splitter 1303 and a polarization beam splitter 1308 via a mirror 1307, and reflected and returned by a reference mirror 1310 via a quarter-wave plate 1309. The light passes through the quarter-wave plate 1309 again, becomes P-polarized light, and enters the polarization beam splitter 1308. On the other hand, the P-polarized light beam that has passed through the polarizing beam splitter 1302 further passes through the polarizing beam splitters 1303 and 1308. Then the light is reflected by a mirror 1311, and is irradiated onto the ocular surface via a quarter-wave plate 1312. The irradiated light beam is reflected on the ocular surface and returns at a frequency of fo+fd by Doppler shift frequency fd due to surface wave vibration. It becomes S-polarized light by the quarter-wave plate 1312 and is reflected by the polarization beam splitter 1308. The light beam from the reference mirror 1310 and the light beam from the ocular surface interfere and enter the photodetector 1313. A signal with a frequency of fm+fd is output from the photodetector 1313, and phase modulation and frequency modulation components are obtained by FM demodulating the modulation component of the Doppler shift frequency fd that changes due to vibration. As a result, surface vibration due to surface waves can be detected.

(5) Arrangement of excitation means and detection means

Efficient excitation can be achieved when the irradiation axis of the excitation means coincides with the normal line of the excitation point against the corneal curved surface. Similarly, the detection sensitivity is high when the detection axis of the detection means also coincides with the normal line against the corneal curved surface. FIG. 14 shows the arrangement of the excitation part 102 and the detection part 103. The alignment is such that the intersection of the irradiation axis of the excitation energy irradiated by the excitation part 102 and the detection axis by the detection part 103 substantially coincides with the center of corneal curvature. When the intersection of the irradiation axis and the detection axis coincide with the center of corneal curvature, the irradiation axis and the detection axis also coincide with the normal line at the excitation point and the detection point. When performing alignment during measurement, the excitation part 102 and detection part 103 are adjusted according to this arrangement.

(6) Processing of obtained data

The data obtained by the detection part 103 of the eyeball property measuring device 1 according to the present embodiment is outputted from the detection part 103 to the surface wave processing part 208 as parameters such as phase, frequency, and amplitude. The surface wave processing part 208 calculates the phase and delay time at the detection point of the surface wave based on these data. Next, the ocular physical property calculation part 209 calculates the phase and the phase change, wherein the phase change can be used for calculating the delay time using the excitation signal from the excitation part 102 as a reference when there is only one detection point. If there are multiple detection points, the phase difference is determined from the vibration waveform obtained from each detection point. The phase velocity is calculated from this phase difference, the frequency, and the distance from the excitation point to the detection point.

Furthermore, the ocular physical property calculation part 209 calculates the physical properties of the eyeball (surface tension, shear modulus, Young's modulus, and viscosity modulus) based on the following equation. Specifically, the following equations (1) and (2) show the relationship between Young's modulus E and shear modulus μ. Therefore, if the shear modulus is calculated from the phase velocity of the surface wave, the Young's modulus can also be calculated.

$$\mu = \frac{E}{2(1+\nu)} \cdots (1)$$

$$E = 2\mu(1 + v) \cdots (2)$$

Herein, $\mu$: shear modulus, E: Young's modulus, $v$: Poisson's ratio

The below-mentioned equation shows the equations for determining the physical properties of the eyeball, such as intraocular pressure and shear modulus, from the phase velocity of the waves depending on the type of surface wave (Rayleigh wave, Rayleigh-Lamb wave, surface tension wave, and leaky surface tension wave). For example, according to Non-Patent reference 2, when the Poisson's ratio of the tissue is 0.49, the phase velocity Cr of the Rayleigh wave is expressed by equation (3). Since the density $\rho$ of the ocular surface tissue is known, the shear modulus $\mu$ can be calculated by measuring Cr using this measurement method.

$$c_r = \frac{0.87 + 1.12v}{1 + v}\sqrt{\frac{\mu}{\rho}} \cdots (3)$$

Herein, Cr: Rayleigh wave phase velocity, $\mu$: shear modulus, $\rho$: density, $v$: Poisson's ratio

The following equations (4), (5), (6), and (7) are known as Rayleigh-Lamb wave equations for the cornea from Non-Patent reference 4, etc. Since the corneal thickness d, corneal density $\rho$, water density $\rho$F, and angular frequency $\omega$ of the surface wave are known, the shear modulus $\mu$ and viscosity modulus $\eta$ can be calculated from the phase velocity of the Rayleigh-Lamb wave measured by this measurement method.

$$M = \begin{bmatrix} (k^2 + \beta^2)\sinh(\alpha d) & 2k\beta\,\sinh(\beta d) & (k^2 + \beta^2)\cosh(\alpha d) & 2k\beta\,\cosh(\beta d) & 0 \\ 2k\alpha\,\cosh(\alpha d) & (k^2 + \beta^2)\cosh(\beta d) & 2k\alpha\,\sinh(\alpha d) & (k^2 + \beta^2)\sinh(\beta d) & 0 \\ -(k^2 + \beta^2)\sinh(\alpha d) & -2k\beta\,\sinh(\beta d) & (k^2 + \beta^2)\cosh(\alpha d) & 2k\beta\,\cosh(\beta d) & \frac{\rho_F\omega^2}{\mu^*} \\ 2k\alpha\,\cosh(\alpha d) & (k^2 + \beta^2)\cosh(\beta d) & -2k\alpha\,\sinh(\alpha d) & -(k^2 + \beta^2)\sinh(\beta d) & 0 \\ \alpha\,\cosh(\alpha d) & k\,\cosh(\beta d) & -\alpha\,\sinh(\alpha d) & -k\,\sinh(\beta d) & -\alpha_F \end{bmatrix}$$

$$\cdots (4)$$

$$\mu^* = \mu + i\omega\eta \cdots (5)$$

$$\alpha_F^2 = k^2 - \frac{\omega^2}{c_F^2} \cdots (6)$$

$$k = \frac{\omega}{c_p} \cdots (7)$$

Herein, Cp: phase velocity of Rayleigh-Lamb wave, $\rho$: corneal density, $\rho$F: water density d: corneal thickness x 1/2, k: wave number of surface wave, $\omega$: angular frequency of surface wave, $\mu$: shear elasticity rate, $\eta$: viscosity coefficient, cF: sound velocity of water

According to Non-Patent reference 1 and so on, Equation (8) shows the relationship between the phase velocity Cc of the surface tension wave and the surface tension $\gamma$. Since the density $\rho$ of the ocular surface tissue and the angular frequency of the surface waves are known, the surface tension can be calculated from the phase velocity of the surface tension waves, wherein the phase velocity can be measured by this measurement method. Equation (10) is based on Laplace's law, and the internal pressure Pi - external pressure Po can be calculated by measuring the surface tension and surface curvature. Herein, the surface curvature is the curvature of the cornea and the external pressure is atmospheric pressure. Therefore, the internal pressure, that is, the intraocular pressure, can be calculated from the surface tension calculated by this measurement method.

$$c_c = \sqrt[3]{\frac{\gamma \omega}{\rho}} \cdots (8)$$

Herein, cc: phase velocity of surface tension wave, $\gamma$: surface tension, $\rho$: density, $\omega$: angular frequency of surface wave

Based on Non-patent reference 2, non-patent reference 3, etc., equation (9) shows the relationship between the phase velocity Clc of a leaky surface tension wave, the surface tension $\gamma$, and the shear modulus $\mu$. The surface tension $\gamma$ can be determined from the density $\rho$, the shear modulus $\mu$, and the phase velocity Clc of the leaky surface tension wave, and the intraocular pressure can be calculated from Laplace's law equation (10).

$$c_{lc} = \sqrt{\frac{\gamma}{\rho}k + 4\frac{\mu}{\rho}} \cdots (9)$$

Herein, Clc: phase velocity of leaky surface tension wave, $\gamma$: surface tension, $\rho$: density, $\mu$: shear modulus

$$P_i - P_o = \frac{2\gamma}{R} \cdots (10)$$

Herein, Pi: internal pressure (intraocular pressure), Po: external pressure (atmospheric pressure), $\gamma$: surface tension, R: surface curvature (corneal curvature)

Herein, relational equations for surface waves in soft viscoelastic materials have been published except for these mentioned expressions, and equations other than the above equations (1) to (10) can also be adopted. The calculation accuracy may vary depending on the adapted equation. In reality, since it is affected by the unique conditions of each organism, such as the heterogeneity of the living body and the size of the excitation point and detection point, it is necessary to use these equations as a basis and find approximate equations using clinical data. For example, in the case of intraocular pressure measurement, a contact-type applanation tonometer is considered to be a relatively accurate and precise measurement for clinical intraocular pressure measurement. This type of tonometer measures intraocular pressure based on the pressure when a certain area is applanated. The above theoretical equation can also be corrected by approximately calculating the relationship between intraocular pressure measurement using these applanation tonometers and the phase velocity of surface tension waves. As for an actual device configuration, it is necessary to arrange a correction equation based on the correlation between surface wave measurement data and clinical data and incorporate it into the calculation unit of the device. Furthermore, by accumulating clinical data, it is also possible to calculate intraocular pressure and material mechanical properties of ocular tissues without using calculation formulas, instead of using techniques such as artificial intelligence (AI) and machine learning.

(7) Embodiment of corneal anterior curvature measurement function

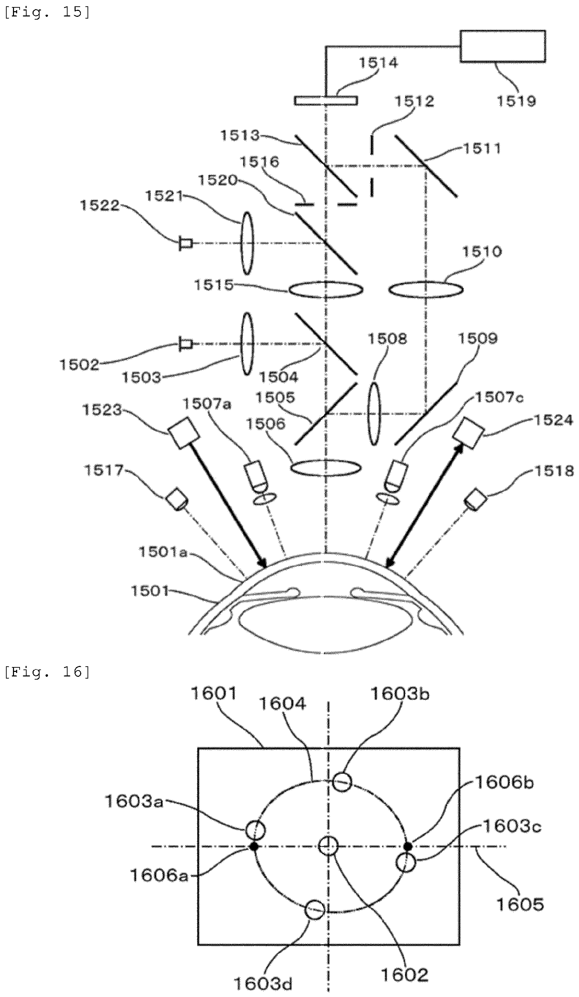

In a case when calculating the intraocular pressure of the eye to be examined from the surface tension calculated from the phase velocity of the surface wave, the curvature of the cornea of the eye to be examined is required. In a case when measuring intraocular pressure, it is possible to calculate the intraocular pressure value of the eye to be examined by measuring the corneal curvature value of the eye in advance using an integrated or separate measuring device and inputting the measured value. However, accuracy can be improved by measuring the corneal curvature along the corneal cross section that includes the excitation point and detection point. Therefore, it is desirable for the ocular physical property measuring device 1 to have a corneal curvature measurement function to measure the corneal curvature at the same time as surface wave measurement. As for this measuring method, the method shown in FIGS. 15 and 16 below can be applied.

As shown in Patent References 6 and 7, the corneal curvature of the subject's eye is measured by calculating the corneal curvature based on the coordinates of a plurality of dot-like indicators formed on the image receiving surface of an image sensor, wherein a plurality of punctate targets are projected onto the corneal surface of the eye to be examined, and the punctate targets are photographed using an imaging optical system for photographing the plurality of punctate targets projected onto the cornea of the eye to be examined. This method is well-known.

In a case when a plurality of punctate targets are projected onto the corneal surface of the eye to be examined and when an image is formed on a two-dimensional image sensor using an imaging optical system, one of the plurality of punctate targets projected onto the cornea of the eye to be examined is coaxial with the optical axis of the imaging optical system for photographing the punctate targets. This is regarded as a first visual target. A plurality of other punctate targets are arranged concentrically around the optical axis of the imaging optical system and projected onto the cornea of the eye to be examined. These are regarded as second visual targets. In a case when the imaging optical system is aligned and photographed so that the first visual target is imaged at the optical axis point of the two-dimensional image sensor of the imaging optical system, the corneal curvature of the eye to be examined can be calculated using the positional relationship of the imaged coordinates on the two-dimensional image sensor between the first visual target and the second visual targets. If there are three or more second visual targets, calculate the imaging position of the second visual targets using an ellipse approximation with the first visual target as the center of the ellipse. Then, based on the calculated elliptical shape, it is possible to measure the curvature of the cornea of the eye to be examined, including the astigmatic component.

If there are two second visual targets, the astigmatism component of the cornea of the eye to be examined cannot be measured. However, the corneal curvature within the cross section can be measured by aligning the two second visual targets on the cornea of the eye to be examined within the same cross section including the excitation axis and the detection axis. Since the corneal curvature of the eye to be examined required for intraocular pressure measurement can be calculated only from the curvature in the cross section including the excitation axis and the detection axis, two of the second visual targets may be used.

FIG. 15 shows an example in which there are four second visual targets. A measurement light source 1502 coaxial with the imaging optical axis and collimated light sources 1507a to 1507d around the imaging optical axis are light sources for projecting punctate targets onto the corneal surface. The light emitted from the light source 1502 is reflected by a half mirror 1504 via a relay lens 1503, and projected onto a cornea 1501a of an eye to be examined 1501 via a dichroic mirror 1505 and an objective lens 1506. Furthermore, parallel light beams emitted from the collimated measurement light sources 1507a to 1507d arranged on concentric circles around the optical axis are projected onto the cornea 1501a of the eye to be examined. The reflected images of each light source reflected from the cornea 1501a of the eye to be examined, are formed on a two-dimensional image sensor 1514 as a punctate image target, via an objective lens 1506, dichroic mirror 1505, half mirror 1504, relay lens 1515, dichroic mirror 1520, aperture 1516 and dichroic mirror 1513.

FIG. 16 shows how the corneal reflection images of the light sources 1502 and 1507a to 1507d are formed as the punctate image targets on the imaging plane 1601 of the two-dimensional image sensor. The reflected images 1602 and 1603a to 1603d correspond to light sources 1502 and 1507a to 1507d in FIG. 15, respectively. Since the light source 1502 is always emitted coaxially with the optical axis of the imaging optical system, if the light sources 1503a to 1503d are arranged symmetrically around the optical axis, 1602 is considered to be the geometric center of the approximate ellipse 1604. As a result, an approximate ellipse 1604 can be determined if the coordinates of three or more punctate target points among the reflected image 1602 and reflected images 1603a to 1603d, are found. A line on the light receiving surface of the two-dimensional image sensor corresponding to the cross section formed by the irradiation axis of the excitation part and the detection axis of the detection part, is defined as a detection line 1605. In this case, the curvature on the detection line can be calculated from the distance between the coordinates of two points 1606a and 1606b wherein the detection line 1605 and the ellipse 1604 intersect at the point 1606a. The above calculation process is processed by the processing unit 1519.

In addition, among the light sources around the imaging optical system, the light irradiation optical axe of the light sources 1507a and 1507c is arranged so as to be in the same plain with the cross section formed by the irradiation axis of the excitation part 1523 and the detection axis of the detection part 1524. In this case, by tarusing the positional coordinates of the reflected images 1602, 1603a, and 1603c formed by the light source 1502, the light source 1507a, and the light source 1507c on a two-dimensional image sensor, the corneal curvature of the same plane as the formed cross section by the irradiation axis of the excitation part and the detection axis of the detection part, can be determined without calculating an approximate ellipse.

In the embodiment shown in FIG. 15, in addition to the measurement optical system for measuring corneal curvature, an anterior segment observation optical system is provided as an optical system for observing the anterior segment of the eye. In this anterior segment observation optical system, reflected and scattered light from the anterior ocular segment illuminated by illumination light sources 1518 and 1519 for anterior ocular segment illumination is reflected by a dichroic mirror 1505, and then passes through relay lens 1508, mirror 1509, relay lens 1510, mirror 1511, and an aperture 1512, is reflected by a dichroic mirror 1513, and is imaged by a two-dimensional image sensor 1514.

Furthermore, a fixation lamp optical system comprising a fixation light source 1522, a relay lens 1521, and a dichroic mirror 1520 for fixating the eye 1501 to be examined during measurement, is provided.

(8) Embodiment of corneal curvature and corneal thickness measurement using light-section method

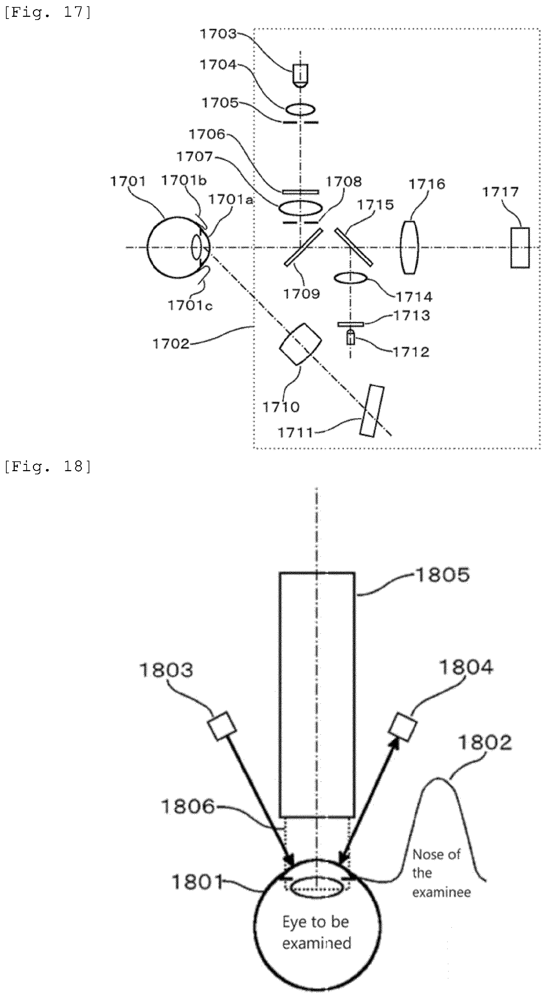

As disclosed in Patent Reference 8 and 9, a method of correcting the intraocular pressure value measured by a conventional air-puff tonometer based on the cornea thickness of the eye to be examined, is known. In a case when calculating the intraocular pressure of the eye to be examined from the surface tension calculated from the phase velocity of the surface wave generated on the corneal surface, it is desirable to consider the influence of the corneal thickness to calculate the intraocular pressure value more accurately. Herein, it is possible to correct the intraocular pressure value of the eye to be examined by measuring the corneal thickness of the eye in advance using an integrated or separate measuring device and inputting the measured value. However, accuracy can be improved by measuring the corneal thickness along the corneal cross section that includes the excitation point and detection point. Therefore, it is desirable for the ocular physical property measuring device 1 to be equipped with a corneal curvature measurement function to measure corneal thickness and corneal curvature at the same time as measuring surface waves. As a measuring method for this, the method shown in FIGS. 17 and 18 below can be applied.

The method of measuring the cross-sectional shape of the cornea using the light-section method is well known, such as from Patent Reference 10, 11, 12, etc. With the light-section method, not only the corneal thickness but also the corneal curvature can be measured at the same time.

This is an embodiment in which the thickness and curvature of the cornea of the eye to be examined are calculated by measuring the corneal shape using a light-section method with a tonometer that measures surface waves generated on the eye to be examined. When measuring the corneal curvature and corneal thickness of the eye to be examined, accuracy can be improved by measuring the corneal curvature along the corneal cross section that includes the excitation point and detection point. Therefore, this embodiment takes that point into consideration. The light-section method includes a slit projection optical system that projects a slit light onto the cornea of the eye to be examined, and a cross-sectional photographing optical system that takes a photo of the light reflected and scattered from the cornea of the eye to be examined from an angle against the slit projected cross section wherein the split light enters the eye to be examined. In this case, the slit projection optical system, the cross-sectional photographing optical system, and the two-dimensional image sensor for recording images are constructed based on the Scheimpflug principle. The photographed cross-sectional image of the cornea of the eye to be examined is corrected for changes in magnification relative to position using the Scheimpflug optical system, and the refractive distortion of the posterior surface of the cornea due to corneal refraction is corrected based on Snell's law. And then, corneal curvature and corneal thickness are calculated by using the anterior cornea surface and calculating the contour of the posterior surface.

FIG. 17 is an embodiment for photographing a cross section of the cornea of the eye to be examined using the Scheimpflug photographing principle of the light-section method. The eye to be examined 1701 is a vertical cross-sectional view of the eye to be examined. 1701a, 1701b, and 1701c shows the cornea of the eye to be examined, the upper eyelid of the eye to be examined, and the lower eyelid of the eye to be examined, respectively. 1702 is the entire optical system for photographing the Scheimpflug of the cornea of the eye to be examined using the light-section method. The cross-sectional imaging light source 1703 is a light source for projecting slit light onto the eye to be examined. The slit light is formed by a condenser lens 1704 and a slit 1705, and the slit light is projected horizontally onto the eye to be examined via a polarizing filter, a slit light projection lens, a rectangular opening aperture 1708, and a polarizing beam splitter. A polarizing filter 1706 and a polarizing beam splitter 1709 prevent the corneal projection image of the slit light from entering the two-dimensional imaging device, wherein the device is for anterior ocular segment observation and alignment.

Therefore, this is designed not to affect the anterior segment observation image and the imaging of an alignment target 1713, which is described below.

A cross-section photographing optical system comprises a cross-section photographing lens 1710 and a two-dimensional image sensor 1711 for cross-section photographing. In this embodiment, the photographing optical axis is arranged below the eye to be examined at an angle of 45 degrees with respect to the projection optical axis of the slit light. The slit projection light cross-section, the main plane of the imaging lens 1710 for cross-sectional photographing, and the extended surface of the light-receiving surface of the two-dimensional image sensor 1711 are arranged so as to intersect at one line of intersection. A Scheimpflug optical system is formed by this.

As an optical system for fixation of the eye to be examined and alignment of the device, a fixation and alignment light source 1712, a fixation and alignment target 1713, and a target projection lens 1714, are arranged. It is projected onto the eye to be examined via a half mirror 1715 and the polarizing beam splitter 1709. The anterior segment image and the alignment target projected onto the eye to be examined are imaged on a two-dimensional imaging device 1717 for anterior segment observation and alignment via the polarizing beam splitter 1709, the half mirror 1715, and the imaging lens 1716.

FIG. 18 shows the arrangement of the cross-sectional photographing optical system for the eye to be examined shown in FIG. 17, the surface wave excitation unit and the surface wave detection unit. FIG. 18 shows a case where the left eye is measured, and an excitation unit 1803 and a detection unit 1804 are arranged for the eye to be examined 1801 and the nose of the examinee 1802. A surface wave is excited on the corneal surface of the eye to be examined, and is detected by the detection unit. And the cross section of the eye to be examined is photographed by the cross-sectional photographing optical system 1805 to measure the thickness and curvature of the cornea. A slit surface 1806 of the projection slit light is formed as shown in the figure, and the excitation axis of the excitation unit 1808 and the detection axis of the detection unit 1804 and the slit surface 1806 are on the same plane. This arrangement makes it possible to measure the corneal thickness and corneal curvature of the eye to be examined along the point from the excitation point to the detection.

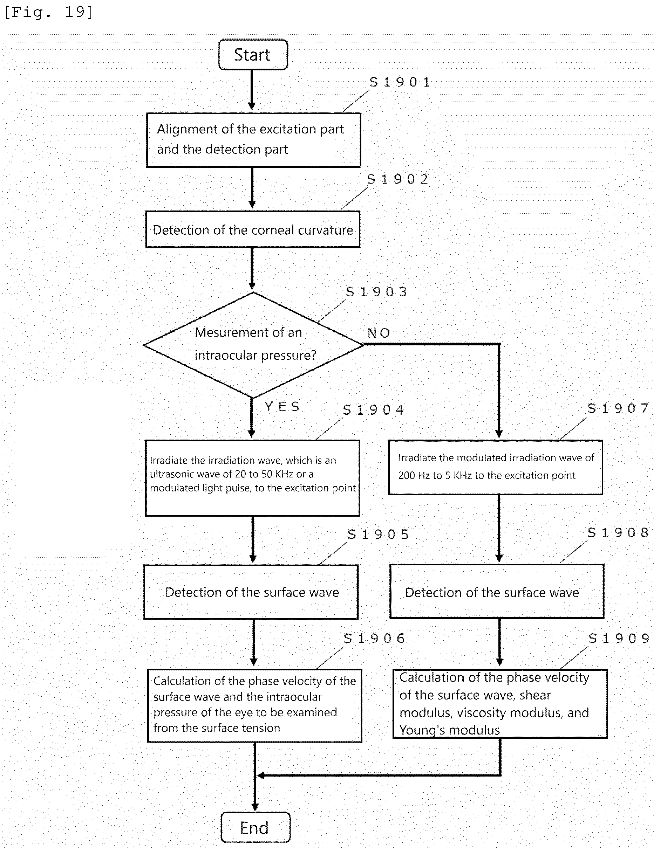

Next, typical operation steps of the ocular physical property measuring device 1 according to the present embodiment will be described by referring to the flowchart shown in FIG. 19. The operation steps is an example in which the excitation point and the detection point are regarded as corneal surface, and the corneal curvature detection device is integrated into the eyeball property measuring device 1 in advance. First, as described above, the excitation part 102 and the detection part 103 are aligned so that the intersection of the irradiation axis 107 of the excitation energy irradiated from the excitation part 102 and the detection axis 108 by the detection part 103 substantially coincides with the center of the corneal curvature (S1901). For alignment, a target projected onto the cornea for corneal curvature measurement can also be used as an alignment target. Then, the corneal curvature of the eye to be examined is determined using the corneal curvature detection device integrated in advance with the ocular physical property measuring device 1 (S1902).

Next, it is determined whether the target of the eye to be examined is intraocular pressure (mainly when surface tension becomes the restoring force of surface waves) or not (S1903). In the case of intraocular pressure (Yes in S1903), an irradiation wave is transmitted from the excitation part 102 toward the excitation point 104 on the eyeball. In this case, the irradiation wave needs to generate a surface tension wave or a leaky surface tension wave as a surface wave on the eyeball. Herein, if the excitation means is ultrasonic waves, since the ultrasonic waves can be transmitted at the same frequency of 20 KHz to 50 KHz as the generation frequency of surface tension waves and leaky surface tension waves, they are irradiated without any modulation. In the case when the excitation means is a light pulse, the modulator 201a is used to amplitude-modulates the high-frequency light pulse by a modulation frequency of 20 to 50 KHz, which is lower than the oscillation frequency, and then the irradiation waves after the modulation are applied to the excitation point 104 on the eyeball (S1904).

Next, the detection part 103 detects surface waves on the eyeball at the detection point 105 (S1905). It is noted that, as this surface wave detection method, for example, an ultrasonic reflection method is used. Then, the surface wave processing unit 208 demodulates the surface wave (in this case, a surface tension wave or a leaky surface tension wave) detected by the detection part 103. The ocular physical property calculation part 209 specifies the phase velocity of this surface wave, calculates the surface tension from this phase velocity using the above (Equation 8) or (Equation 9), and further calculates intraocular pressure from the determined surface tension based on the above mentioned (Equation 10) (S1906). These calculations are corrected using a clinically determined correction formula.

On the other hand, in a case when the measurement target of the eye to be examined is Young's modulus, shear modulus, or viscosity modulus (when shear elasticity is mainly the restoring force of the surface wave) (No in S1903), the irradiation wave is transmitted from the excitation part 102 to the excitation point 104 on the eyeball. In this case, the irradiation wave needs to be a Rayleigh wave or a Rayleigh-Lamb wave as a surface wave on the eyeball. Herein, the modulation part 201a is used to amplitude-modulate the high-frequency ultrasonic waves and optical pulses by a modulation frequency of 200 Hz to 5 KHz, which is lower than the oscillation frequency of the irradiation waves, and then the irradiation waves after the modulation are sent to the excitation point 104 on the eyeball (S1907).

Next, the detection part 103 detects a surface wave on the eyeball at the detection point 105 (S1908). Then, the surface wave processing unit 208 demodulates the surface wave (Rayleigh wave or Rayleigh-Lamb wave in this case) detected by the detection part 103. The ocular physical property calculation unit 209 characterizes the phase velocity of this surface wave, calculates the shear modulus $\mu$ and viscosity modulus $\eta$ from this phase velocity using the above (Equation 3) to (Equation 7), and further calculates Young's modulus from the determined shear modulus $\mu$ using a known calculation formula (S1909).

As mentioned above, the non-contact type ocular physical property measuring device 1 of this embodiment comprises: an excitation part 102 that excites at least one or more excitation points 104 on an eyeball by using an irradiation wave to generate a surface wave on a surface of an eye to be examined; a detection part 103 that detects the surface wave generated by the excitation part 102 at at least one or more detection points 105, which are different from the excitation points 104, on the eyeball; a surface wave processing part 208 that analyzes the surface wave detected by the detection part 103; and an ocular physical property calculation part 209 that calculates physical properties of the eyeball based on the analyze result by the surface wave processing part 208. Herein, the surface wave processing part 208 determines at least one of phase change and delay time of the surface wave. The ocular physical property calculation part 209 determines at least one of phase velocity and group velocity of the surface wave based on at least one of the phase change and delay time of the surface wave and calculates eyeball physical properties based on at least one of the phase velocity and group velocity of the surface wave.

With this configuration, the ocular physical property measuring device 1 according to the present embodiment can use surface waves generated at a predetermined position on the eyeball surface in a non-contact manner, wherein the device 1 can measure the physical properties of the eyeball (intraocular pressure, mechanical material properties of the eyeball tissue, etc.). In other words, the device 1 does not use a device based on vibration or displacement of the eyeball or cornea itself, or a change in phase due to vibration, which are mentioned in conventional methods. Since the intraocular pressure can be measured using surface waves on the eyeball intentionally generated by the excitation part 102, there is no need to vibrate the eyeball itself or the cornea itself. As a result of this, it is possible to measure intraocular pressure, Young's modulus, shear modulus, viscosity, etc. of ocular tissue without the examinee feeling any discomfort or uncomfortable.

Note that in order to achieve the above objects as the ocular physical property measuring device, it is also possible to embody the present invention determining method that includes, as its steps, the characteristic units included in such an ocular physical property measuring method, and as a program causing a computer to execute such steps. It should also be noted that such program can be distributed on a recording medium such as a USB and over a transmission medium such as the Internet.

Reference Signs List

1 ocular physical property measuring device
101 ocular surface
102 excitation part (excitation means)
103 detection part (detection means)
104 excitation point
105 detection point
106 surface wave
107 irradiation axis
108 detection axis
201 excitation unit
201a modulator (modulation means)
202 detection unit
208 surface wave processing part (surface wave processing means)
209 ocular physical property calculation part (ocular physical property calculation means)
210 transmission control part
211 control unit

Claims

1.  A non-contact type ocular physical property measuring device (1), comprising:

an excitation means (102) that excites at least one or more excitation points on an eyeball by using an irradiation wave to generate a surface wave on a surface of an eye to be examined;
a detection means (103) that detects the surface wave generated by the excitation means (102) at at least one or more detection points, which are different from the excitation points, on the eyeball;
a surface wave processing means (208) that analyzes the surface wave detected by the detection means (103); and
an ocular physical property calculation means (209) that calculates physical properties of the eyeball based on the analyze result by the surface wave processing means (208),
wherein the surface wave processing means (208) determines at least one of phase change and delay time of the surface wave;
wherein the ocular physical property calculation means (209) determines at least one of phase velocity and group velocity of the surface wave based on at least one of the phase change and delay time of the surface wave and calculates eyeball physical properties base on at least one of the phase velocity and group velocity of the surface wave **characterized in that** the irradiation wave emitted from the excitation means (102) is a continuous wave of an aerial ultrasonic wave whose basic frequency ranges from 20 KHz to 200 KHz or a burst wave having more than 10 waves of this aerial ultrasonic wave.

2. A non-contact type ocular physical property measuring device (1), comprising:

an excitation means (102) that excites at least one or more excitation points on an eyeball by using an irradiation wave to generate a surface wave on a surface of an eye to be examined;
a detection means (103) that detects the surface wave generated by the excitation means (102) at at least one or more detection points, which are different from the excitation points, on the eyeball;
a surface wave processing means (208) that analyzes the surface wave detected by the detection means (103); and
an ocular physical property calculation means (209) that calculates physical properties of the eyeball based on the analyze result by the surface wave processing means (208), wherein the surface wave processing means (208) determines at least one of phase change and delay time of the surface wave; wherein the ocular physical property calculation means (209) determines at least one of phase velocity and group velocity of the surface wave based on at least one of the phase change and delay time of the surface wave and calculates eyeball physical properties base on at least one of the phase velocity and group velocity of the surface wave, **characterized in that** the irradiation wave emitted from the excitation means (102) is a continuous pulsed light from a coherent light source or a non-coherent light source, and the basic frequency of the continuous pulsed light ranges from 50 KHz to 50 MHz;

the ocular physical property measuring device (1) further comprising:

a modulation means (201a) that amplitude-modulates the pulsed light using a modulation frequency of 200 Hz or more and 100 KHz or less, which is lower than the fundamental frequency of the pulsed light, and
the excitation means (102) continuously irradiates the amplitude-modulated continuous pulsed light obtained by amplitude-modulating the continuous pulsed light at a frequency lower than its fundamental frequency by the modulation means (201a), or irradiates a burst wave having 10 cycles or more of this amplitude-modulated continuous pulsed light.

3. The non-contact type ocular physical property measuring device (1) according to claim 1, further comprising:
a modulation means (201a) that amplitude-modulates the aerial ultrasonic wave using a modulation frequency of 200 Hz or more and 100 KHz or less, which is lower than the fundamental frequency of the aerial ultrasonic wave.

4. The non-contact type ocular physical property measuring device (1) according to claim 2 or 3,

wherein the excitation means (102) generates generate a surface tension wave or a leaky surface tension wave, whose frequency ranges from 20 KHz to 50 KHz in a case when being amplitude-modulated by the modulation means (201a), as a surface wave; and
wherein the ocular physical property calculation means (209) calculates a surface tension, which is the property, based on the phase velocity of the surface tension wave or the leaky surface tension wave, a curvature of the cornea of the eye to be examined measured by an integrated or separate measuring device, and the below (Equation 1) or (Equation 2), and then calculates an intraocular pressure based on the below (Equation 3).

$$\text{(Equation 1)}$$

$$c_c = \sqrt[3]{\frac{\gamma \omega}{\rho}}$$

Herein, $c_c$: phase velocity of surface tension wave, $\gamma$: surface tension, $\rho$: density, $\omega$: angular frequency of surface wave

$$\text{(Equation 2)}$$

$$c_{lc} = \sqrt{\frac{\gamma}{\rho}k + 4\frac{\mu}{\rho}}$$

Herein, Clc: phase velocity of leaky surface tension wave, $\gamma$: surface tension, $\rho$: density, $\mu$: shear modulus

$$(Equation\ 3)$$

$$P_i - P_o = \frac{2\gamma}{R}$$

Herein, Pi: internal pressure (intraocular pressure), Po: external pressure (atmospheric pressure), $\gamma$: surface tension, R: surface curvature (corneal curvature)

**5.** The non-contact type ocular physical property measuring device according to claim 2 or 3,

wherein the excitation means (102) generates Rayleigh wave or Rayleigh-Lamb wave, as a surface wave, whose frequency ranges from 200 Hz to 5KHz in a case when being amplitude-modulate by the modulation means (201a), and
wherein the ocular physical property calculation means (209) calculates shear modulus, Young's modulus, or Poisson's ratio, which is the property, based on a phase velocity of the Rayleigh wave or the Rayleigh-Lamb wave, and the below (Equation 4) to (Equation 6).

$$(Equation\ 4)$$

$$\mu = \frac{E}{2(1+v)}$$

$$E = 2\mu(1+v)$$

Herein, $\mu$: shear modulus, E: Young's modulus, $v$: Poisson's ratio

$$(Equation\ 5)$$

$$c_r = \frac{0.87 + 1.12v}{1+v}\sqrt{\frac{\mu}{\rho}}$$

Herein, Cr: Rayleigh wave phase velocity, $\mu$: shear modulus, $\rho$: density, $v$: Poisson's ratio

(Equation 6)

$$M = \begin{bmatrix} (k^2 + \beta^2)\sinh(\alpha d) & 2k\beta\,\sinh(\beta d) & (k^2 + \beta^2)\cosh(\alpha d) & 2k\beta\,\cosh(\beta d) & 0 \\ 2k\alpha\,\cosh(\alpha d) & (k^2 + \beta^2)\cosh(\beta d) & 2k\alpha\,\sinh(\alpha d) & (k^2 + \beta^2)\sinh(\beta d) & 0 \\ -(k^2 + \beta^2)\sinh(\alpha d) & -2k\beta\,\sinh(\beta d) & (k^2 + \beta^2)\cosh(\alpha d) & 2k\beta\,\cosh(\beta d) & \frac{\rho_F\omega^2}{\mu^*} \\ 2k\alpha\,\cosh(\alpha d) & (k^2 + \beta^2)\cosh(\beta d) & -2k\alpha\,\sinh(\alpha d) & -(k^2 + \beta^2)\sinh(\beta d) & 0 \\ \alpha\,\cosh(\alpha d) & k\,\cosh(\beta d) & -\alpha\,\sinh(\alpha d) & -k\,\sinh(\beta d) & -\alpha_F \end{bmatrix}$$

$$\mu^* = \mu + i\omega\eta$$

$$\alpha_F^2 = k^2 - \frac{\omega^2}{c_F^2}$$

$$k = \frac{\omega}{c_p}$$

Herein, Cp: phase velocity of Rayleigh-Lamb wave, $\rho$: corneal density, $\rho$F: water density d: corneal thickness x 1/2, k: wave number of surface wave, $\omega$: angular frequency of surface wave, $\mu$: shear elasticity rate, $\eta$: viscosity coefficient, cF: sound velocity of water

6. The non-contact type ocular physical property measuring device (1) according to one of claims 1 to 5, wherein the detection means (103) detects a surface wave using one of an ultrasonic reflection method, an optical triangulation method, a confocal method of optical coaxial light beams having multiple wavelengths, a Fourier domain optical interferometer using an optical heterodyne method, or a vibration detection by a laser Doppler interferometer.

7. The non-contact type ocular physical property measuring device (1) according to one of claims 1 to 6, wherein the excitation means (102) and the detection means (103) are arranged such that an intersection of an irradiation axis of the excitation means (102) and a detection axis of the detection means (103) coincides with the center of corneal curvature.

8. A non-contact type ocular physical property measuring method, including:

an excitation step that excites at least one or more excitation points on an eyeball by using an irradiation wave to generate a surface wave on a surface of an eye to be examined;
a detection step that detects the surface wave generated by the excitation step at at least one or more detection points, which are different from the excitation points, on the eyeball;
a surface wave processing step that analyzes the surface wave detected by the detection step; and
an ocular physical property calculation step that calculates physical properties of the eyeball based on the analyze result by the surface wave processing step,
wherein an irradiation wave emitted from the excitation step is a continuous wave of an aerial ultrasonic wave whose basic frequency ranges from 20 KHz to 200 kHz or a burst wave having more than 10 waves of this aerial ultrasonic wave, wherein the surface wave processing step determines at least one of phase change and delay time of the surface wave;
wherein the ocular physical property calculation step determines at least one of phase velocity and group velocity of the surface wave based on at least one of the phase change and delay time of the surface wave and calculates eyeball physical properties base on at least one of the phase velocity and group velocity of the surface wave.

9. A non-contact type ocular physical property measuring method, including:

an excitation step that excites at least one or more excitation points on an eyeball by using an irradiation wave to generate a surface wave on a surface of an eye to be examined;

a detection step that detects the surface wave generated by the excitation step at at least one or more detection points, which are different from the excitation points, on the eyeball;

a surface wave processing step that analyzes the surface wave detected by the detection step; and

an ocular physical property calculation step that calculates physical properties of the eyeball based on the analyze result by the surface wave processing step,

wherein an irradiation wave emitted from the excitation step is a continuous pulsed light from a coherent light source or a non-coherent light source, and the basic frequency of the continuous pulsed light ranges from 50 KHz to 50 MHz;

wherein the ocular physical property measuring device (1) further comprising:

a modulation step that amplitude-modulates the pulsed light using a modulation frequency of 200 Hz or more and 100 KHz or less, which is lower than the fundamental frequency of the pulsed light, and

wherein the excitation step continuously irradiates the amplitude-modulated continuous pulsed light obtained by amplitude-modulating the continuous pulsed light at a frequency lower than its fundamental frequency by the modulation step, or irradiates a burst wave having 10 cycles or more of this amplitude-modulated continuous pulsed light, wherein the surface wave processing step determines at least one of phase change and delay time of the surface wave;

wherein the ocular physical property calculation step determines at least one of phase velocity and group velocity of the surface wave based on at least one of the phase change and delay time of the surface wave and calculates eyeball physical properties base on at least one of the phase velocity and group velocity of the surface wave.

**Patentansprüche**

1. - Berührungslose Messvorrichtung für physikalische Augeneigenschaften (1), umfassend:

eine Erregungseinrichtung (102), die mindestens einen oder mehrere Erregungspunkte auf einem Augapfel durch Verwenden einer Bestrahlungswelle erregt, um eine Oberflächenwelle auf einer Oberfläche eines zu untersuchenden Auges zu erzeugen;

eine Erfassungseinrichtung (103), die die von der Erregungseinrichtung (102) erzeugte Oberflächenwelle an mindestens einem oder mehreren Erfassungspunkten, die sich von den Erregungspunkten unterscheiden, auf dem Augapfel erfasst;

ein Oberflächenwellenverarbeitungseinrichtung (208), die die von der Erfassungseinrichtung (103) erfasste Oberflächenwelle analysiert; und

eine Berechnungseinrichtung für physikalische Augeneigenschaft (209), die die physikalischen Eigenschaften des Augapfels basierend auf dem Analyseresultat durch die Oberflächenwellenverarbeitungseinrichtung (208) berechnet,

wobei die Oberflächenwellenverarbeitungseinrichtung (208) mindestens eines von Phasenänderung und Verzögerungszeit der Oberflächenwelle bestimmt; wobei die Berechnungseinrichtung für physikalische Augeneigenschaft (209) mindestens eines von Phasengeschwindigkeit und der Gruppengeschwindigkeit der Oberflächenwelle basierend auf der Phasenänderung und/oder der Verzögerungszeit der Oberflächenwelle bestimmt und physikalische Augapfeleigenschaften basierend auf mindestens einem von der Phasengeschwindigkeit und der Gruppengeschwindigkeit der Oberflächenwelle berechnet; **dadurch gekennzeichnet, dass** die von den Erregungseinrichtungen (102) emittierte Bestrahlungswelle eine kontinuierliche Welle einer Luft-Ultraschallwelle ist, deren Grundfrequenz in einem Bereich von 20 kHz bis 200 kHz ist, oder eine Burst-Welle, die mehr als 10 Wellen dieser Luft-Ultraschallwelle aufweist.

2. - Berührungslose Messvorrichtung für physikalische Augeneigenschaften (1), umfassend:

eine Erregungseinrichtung (102), die mindestens einen oder mehrere Erregungspunkte auf einem Augapfel durch Verwenden einer Bestrahlungswelle erregt, um eine Oberflächenwelle auf einer Oberfläche eines zu untersuchenden Auges zu erzeugen;

eine Erfassungseinrichtung (103), die die von der Erregungseinrichtung (102) erzeugte Oberflächenwelle an mindestens einem oder mehreren Erfassungspunkten, die sich von den Erregungspunkten unterscheiden, auf dem Augapfel erfasst;

ein Oberflächenwellenverarbeitungseinrichtung (208), die die von der Erfassungseinrichtung (103) erfasste Oberflächenwelle analysiert; und

eine Berechnungseinrichtung für physikalische Augeneigenschaft (209), die die physikalischen Eigenschaften des Augapfels basierend auf dem Analyseresultat durch die Oberflächenwellenverarbeitungseinrichtung (208) berechnet, wobei die Oberflächenwellenverarbeitungseinrichtung (208) mindestens eines von Phasenänderung und Verzögerungszeit der Oberflächenwelle bestimmt; wobei die Berechnungseinrichtung für physikalische Augeneigenschaft (209) mindestens eines von Phasengeschwindigkeit und der Gruppengeschwindigkeit der Oberflächenwelle basierend auf der Phasenänderung und/oder der Verzögerungszeit der Oberflächenwelle bestimmt und die physikalische Augapfeleigenschaften basierend auf mindestens einem von der Phasengeschwindigkeit und der Gruppengeschwindigkeit der Oberflächenwelle berechnet, **dadurch gekennzeichnet, dass** die von der Erregungseinrichtung (102) emittierte Bestrahlungswelle ein kontinuierliches gepulstes Licht aus einer kohärenten Lichtquelle oder einer nicht-kohärenten Lichtquelle ist und die Grundfrequenz des kontinuierlichen gepulsten Lichts in einem Bereich von 50 kHz bis 50 MHz ist;

die Messvorrichtung für physikalische Augeneigenschaft (1) ferner umfassend:

eine Modulationseinrichtung (201a), die das gepulste Licht mit einer Modulationsfrequenz von 200 Hz oder mehr und 100 kHz oder weniger, die niedriger als die Grundfrequenz des gepulsten Lichts ist, amplitudenmoduliert, und

die Erregungseinrichtung (102) kontinuierlich das amplitudenmodulierte kontinuierliche gepulste Licht emittiert, das durch Amplitudenmodulation des kontinuierlichen gepulsten Lichts mit einer Frequenz, die niedriger ist als seine Grundfrequenz, durch die Modulationseinrichtung (201a) erlangt wird, oder eine Burst-Welle, die 10 Zyklen oder mehr dieses amplitudenmodulierten kontinuierlichen gepulsten Lichts aufweist, emittiert.

3. - Berührungslose Messvorrichtung für physikalische Augeneigenschaft (1) nach Anspruch 1, ferner umfassend:

eine Modulationseinrichtung (201a), die die Luft-Ultraschallwelle unter Verwendung einer Modulationsfrequenz von 200 Hz oder mehr und 100 kHz oder weniger, die niedriger ist als die Grundfrequenz der Luft-Ultraschallwelle, amplitudenmoduliert.

4. - Berührungslose Messvorrichtung für physikalische Augeneigenschaft (1) nach Anspruch 2 oder 3,

wobei die Erregungseinrichtung (102) eine Oberflächenspannungswelle oder eine Oberflächenspannungsleckwelle, deren Frequenz in einem Bereich von 20 kHz bis 50 kHz ist, wenn sie durch die Modulationseinrichtung (201a) amplitudenmoduliert wird, als Oberflächenwelle erzeugt; und

wobei die Berechnungseinrichtung für physikalische Augeneigenschaft (209) eine Oberflächenspannung, die die Eigenschaft ist, basierend auf der Phasengeschwindigkeit der Oberflächenspannungswelle oder der Oberflächenspannungsleckwelle, einer Krümmung der Hornhaut des zu untersuchenden Auges, die durch eine integrierte oder separate Messvorrichtung gemessen wird, und der nachstehenden (Gleichung 1) oder (Gleichung 2) berechnet und dann einen Augeninnendruck basierend auf der nachstehenden (Gleichung 3) berechnet.

$$\text{(Gleichung 1)}$$

$$c_c = \sqrt[3]{\frac{\gamma \omega}{\rho}}$$

Hierin, cc: Phasengeschwindigkeit der Oberflächenspannungswelle, $\gamma$: Oberflächenspannung, $\rho$: Dichte, $\omega$: Winkelfrequenz der Oberflächenwelle

(Gleichung 2)

$$c_{lc} = \sqrt{\frac{\gamma}{\rho}k + 4\frac{\mu}{\rho}}$$

Hierin, Clc: Phasengeschwindigkeit der Oberflächenspannungsleckwelle, $\gamma$: Oberflächenspannung, $\rho$: Dichte, $\mu$: Schermodul

(Gleichung 3)

$$P_i - P_o = \frac{2\gamma}{R}$$

Hierin, Pi: Innendruck (Augeninnendruck), Po: Außendruck (Atmosphärendruck), $\gamma$: Oberflächenspannung, R: Oberflächenkrümmung (Hornhautkrümmung).

5. - Berührungslose Messvorrichtung für physikalische Augeneigenschaft nach Anspruch 2 oder 3,

wobei die Erregungseinrichtung (102) eine Rayleigh-Welle oder Rayleigh-Lamb-Welle als Oberflächenwelle erzeugt, deren Frequenz in einem Bereich von 200 Hz bis 5 kHz ist, wenn sie durch die Modulationseinrichtung (201a) amplitudenmoduliert wird, und

wobei die Berechnungseinrichtung für physikalische Augeneigenschaft (209) den Schermodul, den Elastizitätsmodul oder die Poissonzahl berechnet, die die Eigenschaft basierend auf einer Phasengeschwindigkeit der Rayleigh-Welle oder der Rayleigh-Lamb-Welle und der folgenden Gleichungen (Gleichung 4) bis (Gleichung 6) ist.

(Gleichung 4)

$$\mu = \frac{E}{2(1+v)}$$

$$E = 2\mu(1+v)$$

Hierin $\mu$: Schermodul, E: Elastizitätsmodul, v: Poissonzahl

(Gleichung 5)

$$c_r = \frac{0.87 + 1.12v}{1+v}\sqrt{\frac{\mu}{\rho}}$$

Hierin, Cr: Rayleigh-Wellen-Phasengeschwindigkeit, $\mu$: Schermodul, $\rho$: Dichte, v: Poissonzahl

(Gleichung 6)

$$M = \begin{bmatrix} (k^2 + \beta^2)\sinh(\alpha d) & 2k\beta\,\sinh(\beta d) & (k^2 + \beta^2)\cosh(\alpha d) & 2k\beta\,\cosh(\beta d) & 0 \\ 2k\alpha\,\cosh(\alpha d) & (k^2 + \beta^2)\cosh(\beta d) & 2k\alpha\,\sinh(\alpha d) & (k^2 + \beta^2)\sinh(\beta d) & 0 \\ -(k^2 + \beta^2)\sinh(\alpha d) & -2k\beta\,\sinh(\beta d) & (k^2 + \beta^2)\cosh(\alpha d) & 2k\beta\,\cosh(\beta d) & \frac{\rho_F\omega^2}{\mu^*} \\ 2k\alpha\,\cosh(\alpha d) & (k^2 + \beta^2)\cosh(\beta d) & -2k\alpha\,\sinh(\alpha d) & -(k^2 + \beta^2)\sinh(\beta d) & 0 \\ \alpha\cosh(\alpha d) & k\cosh(\beta d) & -\alpha\sinh(\alpha d) & -k\sinh(\beta d) & -\alpha_F \end{bmatrix}.$$

$$\mu^* = \mu + i\omega\eta$$

$$\alpha_F^2 = k^2 - \frac{\omega^2}{c_F^2}$$

$$k = \frac{\omega}{c_p}$$

Hierin, Cp: Phasengeschwindigkeit der Rayleigh-Lamb-Welle, ρ: Hornhautdichte, ρF: Wasserdichte, d: Hornhautstärke x 1/2, k: Wellenzahl der Oberflächenwelle, ω: Kreisfrequenz der Oberflächenwelle, μ: Scherelastizitätsrate, η: Viskositätskoeffizient, cF: Schallgeschwindigkeit von Wasser.

**6.** - Berührungslose Messvorrichtung für physikalische Augeneigenschaft (1) nach Anspruch 1 bis 5, wobei die Erfassungseinrichtung (103) eine Oberflächenwelle unter Verwendung eines Ultraschallreflexionsverfahrens, eines optischen Triangulationsverfahrens, eines konfokalen Verfahrens optischer koaxialer Lichtstrahlen, die mehrere Wellenlängen aufweisen, eines optischen Fourier-Domänen-Interferometers unter Verwendung eines optischen Heterodyn-Verfahrens oder einer Schwingungserfassung durch ein Laser-Doppler-Interferometer erfasst.

**7.** - Berührungslose Messvorrichtung für physikalische Augeneigenschaft (1) nach Anspruch 1 bis 6, wobei die Erregungseinrichtung (102) und die Erfassungseinrichtung (103) angeordnet sind, sodass ein Schnittpunkt einer Bestrahlungsachse der Erregungseinrichtung (102) und einer Erfassungsachse der Erfassungseinrichtung (103) mit der Mitte der Hornhautkrümmung zusammenfällt.

**8.** - Berührungsloses Messverfahren für physikalische Augeneigenschaft, das Folgendes beinhaltet:

einen Erregungsschritt, der mindestens einen oder mehrere Erregungspunkte auf einem Augapfel durch Verwenden einer Bestrahlungswelle erregt, um eine Oberflächenwelle auf einer Oberfläche eines zu untersuchenden Auges zu erzeugen;
einen Erfassungsschritt, der die von dem Erregungsschritt erzeugte Oberflächenwelle an mindestens einem oder mehreren Erfassungspunkten, die sich von den Erregungspunkten unterscheiden, auf dem Augapfel erfasst;
ein Oberflächenwellenverarbeitungsschritt, der die von dem Erfassungsschritt erfasste Oberflächenwelle analysiert; und
einen Berechnungsschritt für physikalische Augeneigenschaft, die die physikalischen Eigenschaften des Augapfels basierend auf dem Analyseresultat durch den Oberflächenwellenverarbeitungsschritt berechnet, wobei eine aus dem Erregungsschritt emittierte Bestrahlungswelle eine kontinuierliche Welle einer Luft-Ultraschallwelle ist, deren Grundfrequenz in einem Bereich von 20 kHz bis 200 kHz ist, oder eine Burst-Welle die mehr als 10 Wellen dieser Luft-Ultraschallwelle aufweist, wobei der Oberflächenwellenverarbeitungsschritt mindestens eines von Phasenänderung und Verzögerungzeit der Oberflächenwelle bestimmt;

wobei der Berechnungsschritt für physikalische Augeneigenschaft mindestens eines von Phasengeschwindigkeit und der Gruppengeschwindigkeit der Oberflächenwelle basierend auf der Phasenänderung und/oder der Verzögerungzeit der Oberflächenwelle bestimmt und physikalische Augapfeleigenschaften basierend auf mindestens einem von der Phasengeschwindigkeit und der Gruppengeschwindigkeit der Oberflächenwelle berechnet.

9. - Berührungsloses Messverfahren für physikalische Augeneigenschaft, das Folgendes beinhaltet:

einen Erregungsschritt, der mindestens einen oder mehrere Erregungspunkte auf einem Augapfel durch Verwenden einer Bestrahlungswelle erregt, um eine Oberflächenwelle auf einer Oberfläche eines zu untersuchenden Auges zu erzeugen;

einen Erfassungsschritt, der die von dem Erregungsschritt erzeugte Oberflächenwelle an mindestens einem oder mehreren Erfassungspunkten, die sich von den Erregungspunkten unterscheiden, auf dem Augapfel erfasst;

ein Oberflächenwellenverarbeitungsschritt, der die von dem Erfassungsschritt erfasste Oberflächenwelle analysiert; und

einen Berechnungsschritt für physikalische Augeneigenschaft, die die physikalischen Eigenschaften des Augapfels basierend auf dem Analyseresultat durch den Oberflächenwellenverarbeitungsschritt berechnet, wobei eine aus dem Erregungsschritt emittierte Bestrahlungswelle ein kontinuierliches gepulstes Licht aus einer kohärenten Lichtquelle oder einer nicht-kohärenten Lichtquelle ist und die Grundfrequenz des kontinuierlichen gepulsten Lichts in einem Bereich von 50 kHz bis 50 MHz ist;

wobei die Messvorrichtung für physikalische Augeneigenschaft (1) ferner Folgendes umfasst:

einen Modulationsschritt, der das gepulste Licht mit einer Modulationsfrequenz von 200 Hz oder mehr und 100 kHz oder weniger, die niedriger ist als die Grundfrequenz des gepulsten Lichts, amplitudenmoduliert, und

wobei der Erregungsschritt kontinuierlich das amplitudenmodulierte kontinuierliche gepulste Licht emittiert, das durch Amplitudenmodulation des kontinuierlichen gepulsten Lichts mit einer Frequenz, die niedriger ist als seine Grundfrequenz, durch den Modulationsschritt erlangt wird, oder eine Burst-Welle, die 10 Zyklen oder mehr dieses amplitudenmodulierten kontinuierlichen gepulsten Lichts aufweist, ausstrahlt, wobei der Oberflächenwellenverarbeitungsschritt mindestens eines von Phasenänderung und Verzögerungzeit der Oberflächenwelle bestimmt;

wobei der Berechnungsschritt für physikalische Augeneigenschaft mindestens eines von Phasengeschwindigkeit und der Gruppengeschwindigkeit der Oberflächenwelle basierend auf der Phasenänderung und/oder der Verzögerungzeit der Oberflächenwelle bestimmt und physikalische Augapfeleigenschaften basierend auf mindestens einem von der Phasengeschwindigkeit und der Gruppengeschwindigkeit der Oberflächenwelle berechnet.

## Revendications

1. - Dispositif de mesure des propriétés physiques oculaires de type sans contact (1), comprenant :

un moyen d'excitation (102) qui excite au moins un ou plusieurs points d'excitation sur un globe oculaire à l'aide d'une onde d'irradiation pour générer une onde de surface sur une surface d'un œil à examiner ;
un moyen de détection (103) qui détecte l'onde de surface générée par le moyen d'excitation (102) à au moins un ou plusieurs points de détection, différents des points d'excitation, sur le globe oculaire ;
un moyen de traitement d'onde de surface (208) qui analyse l'onde de surface détectée par le moyen de détection (103) ; et
un moyen de calcul des propriétés physiques oculaires (209) qui calcule les propriétés physiques du globe oculaire sur la base du résultat d'analyse par le moyen de traitement d'onde de surface (208),
dans lequel le moyen de traitement d'onde de surface (208) détermine au moins l'un du changement de phase et du temps de retard de l'onde de surface ;
le moyen de calcul des propriétés physiques oculaires (209) déterminant au moins l'une de la vitesse de phase et de la vitesse de groupe de l'onde de surface sur la base d'au moins l'un du changement de phase et du temps de retard de l'onde de surface et calculant les propriétés physiques du globe oculaire sur la base d'au moins l'une de la vitesse de phase et de la vitesse de groupe de l'onde de surface,

**caractérisé par le fait que** l'onde d'irradiation émise par le moyen d'excitation (102) est une onde continue d'une onde ultrasonore aérienne dont la fréquence de base se situe dans la plage de 20 KHz à 200 MHz ou une onde en rafale ayant plus de 10 ondes de cette onde ultrasonore aérienne.

2. - Dispositif de mesure des propriétés physiques oculaires du type sans contact (1), comprenant :

un moyen d'excitation (102) qui excite au moins un ou plusieurs points d'excitation sur un globe oculaire à l'aide d'une onde d'irradiation pour générer une onde de surface sur une surface d'un œil à examiner ;
un moyen de détection (103) qui détecte l'onde de surface générée par le moyen d'excitation (102) à au moins un ou plusieurs points de détection, différents des points d'excitation, sur le globe oculaire ;
un moyen de traitement d'onde de surface (208) qui analyse l'onde de surface détectée par le moyen de détection (103) ; et
un moyen de calcul des propriétés physiques oculaires (209) qui calcule les propriétés physiques du globe oculaire sur la base du résultat d'analyse par le moyen de traitement d'onde de surface (208),
le moyen de traitement d'onde de surface (208) déterminant au moins l'un du changement de phase et du temps de retard de l'onde de surface ;
le moyen de calcul des propriétés physiques oculaires (209) déterminant au moins l'une de la vitesse de phase et de la vitesse de groupe de l'onde de surface sur la base d'au moins l'un du changement de phase et du temps de retard de l'onde de surface et calculant les propriétés physiques du globe oculaire sur la base d'au moins l'une de la vitesse de phase et de la vitesse de groupe de l'onde de surface,
**caractérisé par le fait que** l'onde d'irradiation émise par le moyen d'excitation (102) est une lumière pulsée continue provenant d'une source de lumière cohérente ou d'une source de lumière non cohérente, et que la fréquence de base de la lumière pulsée continue se situe dans la plage de 50 kHz à 50 MHz ;
le dispositif de mesure des propriétés physiques oculaires (1) comprenant en outre
un moyen de modulation (201a) qui module en amplitude la lumière pulsée à l'aide dune fréquence de modulation supérieure ou égale à 200 Hz et inférieure ou égale à 100 KHz, qui est inférieure à la fréquence fondamentale de la lumière pulsée, et
le moyen d'excitation (102) irradiant en continu la lumière pulsée continue modulée en amplitude obtenue par la modulation en amplitude de la lumière pulsée continue à une fréquence inférieure à sa fréquence fondamentale par le moyen de modulation (201a), ou irradiant une onde en rafale ayant 10 cycles ou plus de cette lumière pulsée continue modulée en amplitude.

3. - Dispositif de mesure, des propriétés physiques oculaires de type sans contact (1), selon la revendication 1, comprenant en outre :
un moyen de modulation (201a) qui module en amplitude l'onde ultrasonore aérienne à l'aide d'une fréquence de modulation supérieure ou égale à 200 Hz et inférieure ou égale à 100 KHz, qui est inférieure à la fréquence fondamentale de l'onde ultrasonore qui est aérienne.

4. - Dispositif de mesure des propriétés physiques oculaires de type sans contact, selon la revendication 2 ou 3,

dans lequel le moyen d'excitation (102) génère une onde de tension superficielle ou une onde de tension superficielle de fuite, dont la fréquence se situe dans la plage de 20 KHz à 50 KHz dans le cas où elle est modulée en amplitude par le moyen de modulation, en tant qu'onde de surface ; et
dans lequel le moyen de calcul des propriétés physiques oculaires (209) calcule une tension superficielle, qui est la propriété, basée sur la vitesse de phase de l'onde de tension superficielle ou de l'onde de tension superficielle de fuite, une courbure de la cornée de l'œil à examiner mesurée par un dispositif de mesure intégré ou séparé, et (Equation 1) ou (Equation 2) ci-après, et calcule ensuite une pression intraoculaire basée sur l' (Equation 3) ci-après.

(Équation 1)

$$c_c = \sqrt[3]{\frac{\gamma \omega}{\rho}}$$

dans laquelle cc : vitesse de phase de l'onde de tension superficielle, y : tension superficielle, $\rho$ : densité, $\omega$ : fréquence angulaire de l'onde de surface.

(Équation 2)

$$c_{lc} = \sqrt{\frac{\gamma}{\rho}k + 4\frac{\mu}{\rho}}$$

dans laquelle, Clc : vitesse de phase de l'onde de tension de surface de fuite, $\gamma$ : tension de surface, $\rho$ : densité, $\mu$ : module de cisaillement.

(Équation 3)

$$P_i - P_o = \frac{2\gamma}{R}$$

dans laquelle Pi : pression interne (pression intraoculaire), Po : pression externe (pression atmosphérique), y : tension superficielle, R : courbure de la surface (courbure de la cornée).

5. - Dispositif de mesure des propriétés physiques oculaires de type sans contact, selon la revendication 2 ou 3,

dans lequel le moyen d'excitation (102) génère une onde de Rayleigh ou de Rayleigh-Lamb, en tant qu'onde de surface, dont la fréquence se situe dans la plage de 200 Hz à 5KHz dans le cas où elle est modulée en amplitude par le moyen de modulation (201a), et
dans lequel le moyen de calcul des propriétés physiques oculaires (209) calcule le module de cisaillement, le module de Young ou le rapport de Poisson, qui est la propriété, basée sur une vitesse de phase de l'onde de Rayleigh ou de l'onde de Rayleigh-Lamb, et l'(Equation 4) à l'(Equation 6) ci-après.

(Équation 4)

$$\mu = \frac{E}{2(1 + v)}$$

$$E = 2\mu(1 + v)$$

dans laquelle, $\mu$ : module de cisaillement, E : module d'Young, v : le coefficient de Poisson

(Équation 5)

$$c_r = \frac{0.87 + 1.12\nu}{1 + \nu}\sqrt{\frac{\mu}{\rho}}$$

dans laquelle, Cr : vitesse de phase de l'onde de Rayleigh, $\mu$ : module de cisaillement, $\rho$ : densité, v : le coefficient de Poisson

(Équation 6)

$$M = \begin{bmatrix} (k^2 + \beta^2)\sinh(\alpha d) & 2k\beta\sinh(\beta d) & (k^2 + \beta^2)\cosh(\alpha d) & 2k\beta\cosh(\beta d) & 0 \\ 2k\alpha\cosh(\alpha d) & (k^2 + \beta^2)\cosh(\beta d) & 2k\alpha\sinh(\alpha d) & (k^2 + \beta^2)\sinh(\beta d) & 0 \\ -(k^2 + \beta^2)\sinh(\alpha d) & -2k\beta\sinh(\beta d) & (k^2 + \beta^2)\cosh(\alpha d) & 2k\beta\cosh(\beta d) & \frac{\rho_F \omega^2}{\mu^*} \\ 2k\alpha\cosh(\alpha d) & (k^2 + \beta^2)\cosh(\beta d) & -2k\alpha\sinh(\alpha d) & -(k^2 + \beta^2)\sinh(\beta d) & 0 \\ \alpha\cosh(\alpha d) & k\cosh(\beta d) & -\alpha\sinh(\alpha d) & -k\sinh(\beta d) & -\alpha_F \end{bmatrix} \cdot$$

$$\mu^* = \mu + i\omega\eta$$

$$\alpha_F^2 = k^2 - \frac{\omega^2}{c_F^2}$$

$$k = \frac{\omega}{c_p}$$

dans laquelle Cp : vitesse de phase de l'onde de Rayleigh-Lamb, $\rho$ : densité de la cornée, $\rho$F : densité de l'eau d : épaisseur de la cornée x 1/2, k : nombre d'onde de l'onde de surface, $\omega$ : fréquence angulaire de l'onde de surface, $\mu$ : taux d'élasticité au cisaillement, $\eta$ : coefficient de viscosité, cF : vitesse du son dans l'eau.

6. - Dispositif de mesure des propriétés physiques oculaires de type sans contact, selon l'une des revendications 1 à 5, dans lequel le moyen de détection (103) détecte une onde de surface à l'aide de l'une d'une méthode de réflexion ultrasonore, d'une méthode de triangulation optique, d'une méthode confocale de faisceaux de lumière coaxiaux optiques ayant plusieurs longueurs d'onde, un interféromètre optique à domaine de Fourier utilisant une méthode hétérodyne optique, ou une détection de vibration par un interféromètre laser à effet Doppler.

7. - Dispositif de mesure des propriétés physiques oculaires de type sans contact, selon l'une des revendications 1 à 6, dans lequel le moyen d'excitation (102) et le moyen de détection (103) sont disposés de telle sorte qu'une intersection d'un axe d'irradiation du moyen d'excitation (102) et d'un axe de détection du moyen de détection (103) coïncide avec le centre de courbure de la cornée.

8. - Procédé de mesure des propriétés physiques occulaires de type sans contact, comprenant :

une étape d'excitation qui excite au moins un ou plusieurs points d'excitation sur un globe oculaire à l'aide d'une onde d'irradiation pour générer une onde de surface sur une surface d'un œil à examiner ;

une étape de détection qui détecte l'onde de surface générée par l'étape d'excitation à au moins un ou plusieurs points de détection, qui sont différents des points d'excitation, sur le globe oculaire ;

une étape de traitement d'onde de surface qui analyse l'onde de surface détectée par l'étape de détection ; et

une étape de calcul des propriétés physiques oculaires qui calcule les propriétés physiques du globe oculaire sur la base du résultat d'analyse par l'étape de traitement des ondes de surface,

dans lequel une onde d'irradiation émise par l'étape d'excitation est une onde continue d'une onde ultrasonore aérienne dont la fréquence de base se situe dans la plage de 20 KHz à 200 kHz ou d'une onde en rafale ayant plus de 10 ondes de cette onde ultrasonore aérienne, l'étape de traitement d'onde de surface déterminant au moins l'un du changement de phase et du temps de retard de l'onde de surface ;

dans lequel l'étape de calcul des propriétés physiques oculaires détermine au moins l'une de la vitesse de phase et de la vitesse de groupe de l'onde de surface sur la base d'au moins l'un du changement de phase et du temps de retard de l'onde de surface et calcule les propriétés physiques du globe oculaire sur la base d'au moins l'une de la vitesse de phase et de la vitesse de groupe de l'onde de surface.

**9.** - Procédé de mesure des propriétés physiques oculaires de type sans contact, comprenant

une étape d'excitation qui excite au moins un ou plusieurs points d'excitation sur un globe oculaire à l'aide d'une onde d'irradiation pour générer une onde de surface sur une surface d'un œil à examiner ;

une étape de détection qui détecte l'onde de surface générée par l'étape d'excitation à au moins un ou plusieurs points de détection, qui sont différents des points d'excitation, sur le globe oculaire ;

une étape de traitement d'onde de surface qui analyse l'onde de surface détectée par l'étape de détection ; et

une étape de calcul des propriétés physiques oculaires qui calcule les propriétés physiques du globe oculaire sur la base du résultat d'analyse de l'étape de traitement d'onde de surface,

dans lequel une onde d'irradiation émise par l'étape d'excitation est une lumière pulsée continue provenant d'une source de lumière cohérente ou d'une source de lumière non cohérente, et la fréquence de base de la lumière pulsée continue se situe dans la plage de 50 kHz à 50 MHz ;

dans lequel le dispositif de mesure des propriétés physiques oculaires (1) comprend en outre :

une étape de modulation qui module en amplitude la lumière pulsée à l'aide d'une fréquence de modulation supérieure ou égale à 200 Hz et inférieure ou égale à 100 KHz, qui est inférieure à la fréquence fondamentale de la lumière pulsée, et.

dans lequel l'étape d'excitation irradie en continu la lumière pulsée continue modulée en amplitude obtenue par modulation en amplitude de la lumière pulsée continue à une fréquence inférieure à sa fréquence fondamentale par l'étape de modulation, ou irradie une onde en rafale ayant 10 cycles ou plus de cette lumière pulsée continue modulée en amplitude,

dans lequel l'étape de traitement d'onde de surface détermine au moins l'un du changement de phase et du temps de retard de l'onde de surface ;

dans lequel l'étape de calcul des propriétés physiques oculaires détermine au moins l'une de la vitesse de phase et de la vitesse de groupe de l'onde de surface sur la base d'au moins l'un du changement de phase et du temps de retard de l'onde de surface et calcule les propriétés physiques du globe oculaire sur la base d'au moins l'une de la vitesse de phase et de la vitesse de groupe de l'onde de surface.

[Fig. 1]

Center of corneal curvature

[Fig. 2]

Ocular physical property measuring device 1

Excitation part 102

Detection part 103

201a

Modulator

201

Drive circuit — 203

Transmitting circuit — 204

Receiving circuit — 205

202

Transmission signal generation circuit — 206

A/D convertor — 207

Transmission control part — 210

Surface wave processing part — 208

Ocular physical property calculation part — 209

Control unit — 211

Surface wave

[Fig. 3]

Ultrasonic wave
excitation frequency

$\otimes$

Modulated signal

Modulated ultrasonic
wave waveform

[Fig. 4]

401

F
Focal point control
Mc

Modulated frequency
sound pressure wave

Modulated wave

Mw

[Fig. 5]

501

Ocular surface

Pulsed light

Surface wave

Ultrasonic wave

Hp

[Fig. 6]

Basic pulsed frequency

Modulated frequency signal

Modulated plused light

[Fig. 7]

102

201b                                          201a

                                                          Output modulated light

CW laser  ➡  Modulator  ▪ ▪ ▪ ➡

                          ⬆

                    Controller        Indirectly turning
                                      CW laser ON/OFF
                      201c

[Fig. 8]

102        201d

                          Output modulated light

        LD  ▪ ▪ ▪ ▪ ➡

          ⬆
Current modulated signal

    Current control part   Directly turing semiconductor
                           laser ON/OFF
          201e

[Fig. 9]

[Fig. 10]

[Fig. 11]

Light source

Chromatic aberration focusing lens

Wave length A

Wave length B

Wave length C

Ocular surface

Imaging lens C

Imaging lens B

Imaging lens A

[Fig. 12]

Ocular surface

[Fig. 13]

[Fig. 14]

[Fig. 15]

[Fig. 16]

[Fig. 17]

[Fig. 18]

[Fig. 19]

Start

S1901

Alignment of the excitation part and the detection part

S1902

Detection of the corneal curvature

S1903

Mesurement of an intraocular pressure?

NO

YES

S1904

Irradiate the irradiation wave, which is an ultrasonic wave of 20 to 50 KHz or a modulated light pulse, to the excitation point

S1907

Irradiate the modulated irradiation wave of 200 Hz to 5 KHz to the excitation point

S1905

Detection of the surface wave

S1908

Detection of the surface wave

S1906

Calculation of the phase velocity of the surface wave and the intraocular pressure of the eye to be examined from the surface tension

S1909

Calculation of the phase velocity of the surface wave, shear modulus, viscosity modulus, and Young's modulus

End

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7909765 B
- JP 5314090 B
- JP 2020005679 A
- JP 6289040 B
- JP 5505684 B
- JP 61008592 A
- JP H0360629 A
- JP H08507463 B
- JP 2000060801 A
- JP H0659272 A
- JP 2011050445 A
- JP 2012005835 A
- US 2003078486 A
- US 5148807 A
- US 2018193194 A

**Non-patent literature cited in the description**

- **FRANCISCO MONROY ; DOMINIQUE LANGEVIN**. Direct Experimental Observation of the Crossover from Capillary to Elastic Surface Wave on Soft Gels. *Physical Review Letters*, October 1998, vol. 81 (15)
- **Y. ONODERA ; P. K CHOI**. Surface-wave modes on soft gels. *The Journal of the Acoustical Society of America*, 1998
- **CHOI BOKUN**. Investigating soft materials using surface waves. *Journal, the Acoustical Society of Japan*, 2000, vol. 56 (6), 445-450
- **ZHAOLONG HAN**. Optical coherence elastography assessment of corneal viscoelasticity with a modified Rayleigh-Lamb wave model. *Journal of the Mechanical Behavior of Biomedical Materials*, 2017, vol. 66, 87-94